(19) Europäisches Patentamt
European Patent Office
Office européen des brevets

(11) EP 4 172 170 B1

(12) EUROPEAN PATENT SPECIFICATION

(45) Date of publication and mention of the grant of the patent:
**25.09.2024 Bulletin 2024/39**

(21) Application number: **21734353.2**

(22) Date of filing: **24.06.2021**

(51) International Patent Classification (IPC):
***C07H 19/24*** *(2006.01)* ***C07H 1/00*** *(2006.01)*
***C07H 21/02*** *(2006.01)* ***C12N 15/10*** *(2006.01)*
***C12Q 1/68*** *(2018.01)* ***G01N 33/58*** *(2006.01)*

(52) Cooperative Patent Classification (CPC):
(C-Sets available)
**C07H 19/24; C07H 1/00; C07H 21/02;**
**G01N 33/582;** C12Q 1/6809; Y02P 20/55 (Cont.)

(86) International application number:
**PCT/EP2021/067342**

(87) International publication number:
**WO 2021/260107 (30.12.2021 Gazette 2021/52)**

(54) **FLUORESCENT CYTOSINE ANALOGUES AND THEIR APPLICATION IN TRANSCRIPTION AND TRANSLATION**

CYTOSIN-FLUORESZENZANALOGA UND IHRE ANWENDUNG BEI TRANSKRIPTION UND TRANSLATION

ANALOGUES DE CYTOSINE FLUORESCENTS ET LEUR APPLICATION DANS LA TRANSCRIPTION ET LA TRADUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2020 GB 202009705**

(43) Date of publication of application:
**03.05.2023 Bulletin 2023/18**

(73) Proprietor: **STEALTH LABELS BIOTECH AB**
**412 96 Gothenburg (Västra Götaland County) (SE)**

(72) Inventors:
- **WILHELMSSON, Marcus**
  **412 96 Gothenburg (SE)**
- **ESBJÖRNER, Elin**
  **412 96 Gothenburg (SE)**
- **BALADI, Tom**
  **412 96 Gothenburg (SE)**

(74) Representative: **Alembia Intellectual Property Limited**
**Biohub at Alderley Park**
**Alderley Park**
**Macclesfield, Cheshire SK10 4TG (GB)**

(56) References cited:
**WO-A1-2011/034895 WO-A1-2018/089582**

- **BALADI TOM ET AL: "Stealth Fluorescence Labeling for Live Microscopy Imaging of mRNA Delivery", JOURNAL OF THE AMERICAN CHEMICAL SOCIETY, vol. 143, no. 14, 2 April 2021 (2021-04-02), pages 5413 - 5424, XP055877467, ISSN: 0002-7863, Retrieved from the Internet <URL:https://pubs.acs.org/doi/pdf/10.1021/jacs.1c00014> DOI: 10.1021/jacs.1c00014**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
C12Q 1/6809, C12Q 2521/119, C12Q 2525/101,
C12Q 2563/107

## Description

FIELD

This specification relates to a modified fluorescent nucleobase triphosphate that can be used to biosynthetically produce labelled RNA, which is in turn amenable to in cell translation. This enables live cell imaging in which the labelled messenger RNA and its corresponding translation product (when a fluorescent fusion protein) can be visualised simultaneously.

BACKGROUND

RNA plays a fundamental role in human biology. It is the main player of the central dogma of biochemistry and a crucial regulator of gene expression *via* for instance micro and small interfering RNA, as well as through its intrinsic catalytic activity. It has, for these reasons, also emerged as a highly promising and versatile new drug modality: since RNA therapeutics have the potential to modify cellular function at the translational level, they may open up new opportunities to address previously undruggable targets.

An increased molecular and mechanistic knowledge of the biological processes involving RNA is therefore vital to understanding diseases and treat them. For example, there is a growing body of evidence suggesting that the key to unleashing the full potential of RNA-based drugs lies in understanding the processes of cell uptake and endosomal release (Dowdy, S. F., Nat. Biotechnol. 35, 222-229, [2017]). Regardless of the endocytosis mechanism, the delivery of a nucleic acid cargo to the cytoplasm always relies on endosomal escape, the understanding of which, despite extensive investigations, remains elusive (Crooke, S. T. et al., Not . Biotechnol. 35, 230 [2017]; Pei, D. & Buyanova, M., Bioconjugate Chem. [2018]). In this context, tracking of endogenous and exogenous (therapeutic) RNAs inside cells, including their translocation, localization, splicing and degradation, is of great importance.

Recent advances have resulted in the development of a broad spectrum of tools and probes by which RNA can be analysed and quantified, but they generally involve heavily modified oligonucleotides with properties significantly different from natural ones, potentially resulting in loss of ability to be recognized and processed by the enzymatic machinery of cells. For example, a drawback of existing fluorescence-based technologies for studying cellular localization of RNA is that they primarily rely on highly amphiphilic and/or bulky external fluorescent constructs which could impair motility and perturb localization of the RNA and its molecular interactions with (for example) membrane constituents. In addition, a majority of these technologies are incompatible with live cell imaging (Li, Y., Ke, K. & Spitale, R. C., Biochemistry 58, 379-386 [2019]).

To overcome these issues and provide an improved method of investigating RNA mechanisms, this specification discloses a modified nucleobase triphosphate (compound (I), "$tC^{0}TP$") which can be used to incorporate minimally perturbing and internal labels ("$tC^{0}$") into functional messenger RNA ("mRNA"), giving it utility in live cell imaging and for drug delivery studies. Once incorporated into RNA, the structure of $tC^{O}$ enables it to retain base-pairing and stacking, so that it minimally perturbs natural biological processes (Fig. 1, where the top schematic structure is $tC^{0}$ labelled RNA compared to a conventional externally labelled RNA below). Therefore, this fluorophore constitutes a native-like alternative label, opening new possibilities not only to track the nucleic acid of interest but also to use fluorescent read-outs to obtain detailed information regarding nucleic acid structure and behaviour.

To exemplify the possible applications of $tC^{O}$, the specification describes successful in vitro transcription and also effective in cell translation of a full-length mRNA internally labelled with this fluorescent nucleobase analogue. In addition, by using a transcript encoding for the histone protein H2B fused to a Green Fluorescent Protein ("H2B:GFP") which localizes to the nucleus, the specification shows that it is possible to visualize the labelled mRNA transcripts inside cells while concomitantly recording the fluorescence emanating from the expressed H2B:GFP protein. This approach should be generally applicable to any fluorescent protein.

Wilhelmsson, M. et al. Sci. Rep. 7, 2393 [2017**]** reports the preparation of RNA molecules labelled with $tC^{O}$, but only discloses very short fluorescent RNA oligomers prepared by non-enzymatic solid-phase oligonucleotide synthesis, as opposed to full length RNAs accessible by biosynthesis. By virtue of their method of preparation the labelled RNA molecules are not amenable to in cell "live" analysis of transcription, translation or delivery of long therapeutic RNAs (which are mRNA-based) and therefore do not allow the same level of mechanistic insight.

WO2011/034895 concerns methods for labelling DNA and RNA. It mentions a structurally different fluorescent ribonucleotide analogue 1,3-diaza-2-oxophenothiazine-ribose-5'-triphosphate ("tCTP") which is used during in vitro transcription reactions to prepare labelled RNA. However, unlike the present specification only non-coding labelled RNA was prepared and there is no disclosure of any in cell biosynthesis or visualisation.

The differently labelled, full-length coding RNA polymers accessible using the technology disclosed in the present specification also have advantageous properties over the labelled RNAs in WO2011/034895, for example 1) improved fluorescence levels and label photostability; 2) improved in vitro transcription fidelity; and 3) native-like levels

of in cell translation of $tC^{O}$-labelled mRNA resulting in the correct protein product and localization.

In summary therefore, this specification discloses a labelling technique that not only allows localisation and tracking of the tagged RNA, but also facilitates analysis of the biological functionality and delivery efficacy of mRNA, an important future drug modality. Since the internal $tC^{O}$ label is compatible with biological processes that RNA participates in it holds a great potential to be used as a powerful imaging tool in live cell microscopy, for example in detailed investigations of cellular uptake, endosomal release, exosomal loading and trafficking. These have significant potential to elucidate how these vital delivery pathways work and can be controlled.

## SUMMARY

A primary objective of the present specification is to provide a modified nucleobase triphosphate that can be used to make labelled RNA especially suitable for in vitro and in vivo mechanistic investigations.

Accordingly, this specification describes, in part, a compound of formula (I) or a salt thereof as claimed in claim 1.

This specification also describes, in part, a process for preparing a compound of formula (I) or a salt thereof as claimed in claim 5.

This specification also describes, in part, a composition for preparing a $tC^{O}$ labelled RNA molecule comprising a compound of formula (I) as claimed in claim 16.

This specification also describes, in part, the use of a compound of formula (I) or a salt thereof to enzymatically prepare a $tC^{O}$ labelled RNA molecule as claimed in claim 17.

This specification also describes, in part, a process for preparing a $tC^{O}$ labelled RNA molecule as claimed in claim 19.

This specification also describes, in part, the use of a $tC^{O}$ labelled mRNA molecule to prepare a protein encoded by the mRNA by translation as claimed in claim 20.

## ILLUSTRATIVE EMBODIMENTS

The invention detailed in this specification should not be interpreted as being limited to any of the recited embodiments or examples. Other embodiments will be readily apparent to a reader skilled in the art.

### *General Definitions*

"A" or "an" mean "at least one". In any embodiment where "a" or "an" are used to denote a given material or element, "a" or "an" may mean one. In any embodiment where "a" or "an" are used to denote a given material or element, "a" or "an" may mean 1, 2, 3, 4, 5, 6, 7, 8, 9, 10, 100, 1000, 10000, 100000 or 1000000 (1 million).

When an embodiment includes "a" or "an" feature X, subsequent referrals to "the" feature X do not imply only one of the feature is present. Instead the above interpretation of "a" or "an" continues to apply so that "the" also means "at least one". In other words, embodiments comprising "a feature X, where the feature X is..." should be construed as "at least one feature X, where the at least one feature X is...".

"Comprising" means that a given embodiment may contain other features. For example, in any embodiment where a material "comprising" certain materials or elements is mentioned, the given material may be formed of at least 10% w/w, at least 20% w/w, at least 30% w/w, or at least 40% w/w of the materials or elements (or combination of materials or elements).

In any embodiment where "comprising" is mentioned, "comprising" may also mean "consisting of" (or "consists of") or "consisting essentially of" (or "consists essentially of").

With respect to embodiments of a material, "consisting of" or "consists of" means the material or element is formed entirely of the material or element (or combination of materials or elements). In any embodiment where "consisting of" or "consists of" is mentioned the given material or element may be formed of 100% w/w of the material or element.

With respect to embodiments of a material, "consisting essentially of" or "consists essentially of" means that a given material or element consists almost entirely of that material or element (or combination of materials or elements). In any embodiment where "consisting essentially of" or "consists essentially of" is mentioned the given material or element may be formed of at least 50% w/w, at least 60% w/w, at least 70% w/w, at least 80% w/w, at least 90% w/w, at least 95% w/w or at least 99% w/w of the material or element.

In any embodiment where "is" or "may be" is used to define a material or element, "is" or "may be" may mean the material or element "consists of" or "consists essentially of" the material or element.

When it is mentioned that "in some embodiments..." a certain element may be present, the element may be present in a suitable embodiment in any part of the specification, not just a suitable embodiment in the same section or textual region of the specification.

When a feature is "selected from" a list, the feature is selected from a list consisting of the specified alternatives

(i.e. a list of the alternatives specified and no others).

Claims are embodiments.

*Modified Nucleobase Triphosphates*

In one embodiment there is provided a compound of formula (I) or a salt thereof:

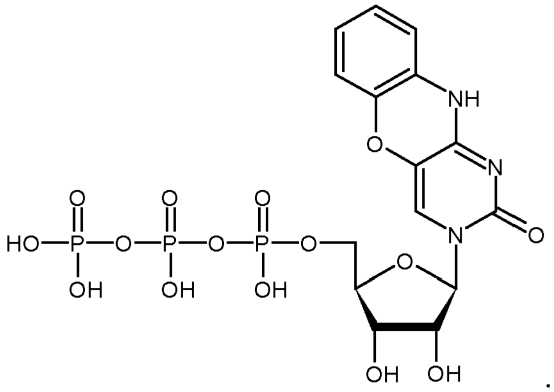

(I)

Compounds and salts described in this specification may exist as a mixture of tautomers (structural isomers resulting from the migration of a hydrogen atom that exist in equilibrium). Relevant embodiments include all tautomers of compounds of formula (I) or salts thereof.

Atoms of the compounds and salts described in this specification may exist as their isotopes. Embodiments include all compounds of formula (I) where an atom is replaced by one or more of its isotopes (for example a compound of formula (I) where one or more carbon atom is an $^{11}C$ or $^{13}C$ carbon isotope, or where one or more hydrogen atom is a $^{2}H$ or $^{3}H$ isotope).

A suitable salt of a compound of formula (I) is for example a base-addition salt. A base-addition salt is formed by bringing the compound of formula (I) into contact with a suitable organic or inorganic base. A base addition salt may be formed using a suitable organic base like a nitrogen base, for example ammonia or a trialkylamine like triethylamine. A base addition salt may also for example be formed using a suitable inorganic base like an alkali metal or rare earth hydroxide, for example potassium hydroxide, sodium hydroxide, magnesium hydroxide or manganese hydroxide.

In one embodiment there is provided a compound of formula (I) which is a free acid.

In one embodiment there is provided a compound of formula (I) which is a salt.

In one embodiment there is provided a compound of formula (I) which is a sodium, potassium, magnesium, or ammonium salt.

In one embodiment there is provided a compound of formula (I) which is a sodium, potassium, or ammonium salt.

In one embodiment there is provided a compound of formula (I) which is a sodium or ammonium salt.

In one embodiment there is provided a compound of formula (I) which is a monosodium, disodium, trisodium, tetrasodium, monoammonium, diammonium, triammonium or tetraammonium salt.

In one embodiment there is provided a compound of formula (I) which is a monosodium, disodium, trisodium or tetrasodium salt.

In one embodiment there is provided a compound of formula (I) which is a monosodium salt.

In one embodiment there is provided a compound of formula (I) which is a disodium salt.

In one embodiment there is provided a compound of formula (I) which is a trisodium salt.

In one embodiment there is provided a compound of formula (I) which is a monoammonium, diammonium, triammonium or tetraammonium salt.

In one embodiment there is provided a compound of formula (I) which is a monoammonium salt.

In one embodiment there is provided a compound of formula (I) which is a diammonum salt.

In one embodiment there is provided a compound of formula (I) which is a triammonium salt.

*Synthetic Processes*

In one embodiment there is provided a process for preparing a compound of formula (I) or a salt thereof comprising:

i. Providing a compound of formula (II) or a salt thereof:

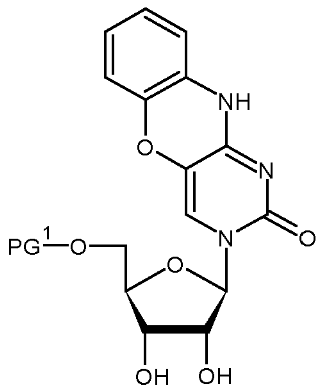

(II)

Where $PG^1$ is a suitable protecting group;
ii. Immobilising the compound of formula (II) or a salt thereof by linking one of its secondary alcohol groups to a suitable support;
iii. Capping any remaining secondary alcohol groups with a suitable protecting group $PG^2$;
iv. Removing the protecting group $PG^1$;
v. Reacting the exposed primary alcohol group with a compound of formula (III):

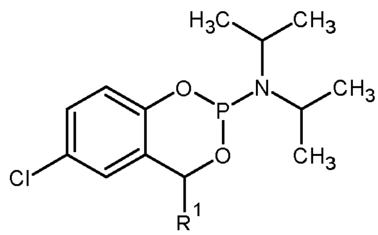

(III)

Where $R^1$ is selected from a hydro group and a $C_{1-3}$alkyl group;
vi. Oxidising the resultant phosphorus (III) compound to a phosphorus (V) compound;
vii. Reacting the phosphorus (V) compound with a tetraalkylammonium pyrophosphate to generate a triphosphate;
viii. Removing the protecting group $PG^2$; and
ix. Cleaving the resultant triphosphate from the support to generate a compound of formula (I) or salt thereof.

A protecting group ("PG", for example $PG^1$ and $PG^2$) is any group suitable for temporarily protecting a reactive centre, for example a hydroxyl group. Suitable protecting groups for the reactive centres disclosed herein may be found for example in "Greene's Protective Groups in Organic Synthesis, Fourth Edition", Greene T. W., Wuts P. G. M.; John Wiley & Sons, Inc. 2007, doi: 10.1002/0470053488).
A "hydro" group is equivalent to a hydrogen atom. Atoms with a hydro group attached to them can be regarded as unsubstituted.
A "$C_{1-3}$alkyl group" is a straight chain or branched saturated alkyl group with the indicated number of carbons. Example $C_{1-3}$alkyl groups include methyl, ethyl, propyl and isopropyl.
In step iii) above, the secondary alcohols to be capped may be those on the ribose part of the molecule.
This overall process is an advantageous preparation of the compound of formula (I) for several reasons:

- There is no need for prior protection of the secondary alcohol groups on the starting protected nucleoside;
- After step 1, unreacted nucleoside can be recovered, which minimizes loss of material;
- The solid-supported nucleosides can be stored for up to 3 months without degradation;
- The route is compatible with automated synthesis;
- The process is robust and has good overall reproducibility;
- Clean phosphorylation crude products are generated that are easy to purify; and

- Gives high phosphorylation yields (typically *ca*. 60 % starting from the nucleoside loaded resin).

In some embodiments $R^1$ may be a hydro group.

In some embodiments $R^1$ may be a $C_{1-3}$alkyl group. It has been observed that when $R^1$ is a $C_{1-3}$alkyl group, the phosphoramidite reagent preparation is easier and higher yielding, but performs at least as well in step v above as when $R^1$ is a hydro group.

In some embodiments $R^1$ may be methyl.

In one embodiment there is provided a compound of formula (III):

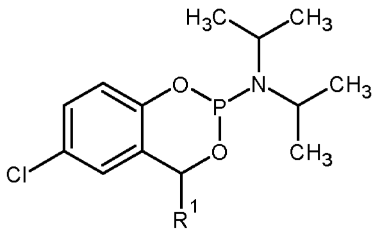

(III)

Where $R^1$ is a $C_{1-3}$ alkyl group.

In one embodiment there is provided a compound of formula (IIIa):

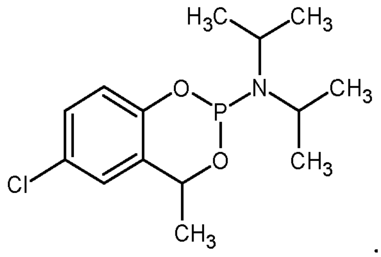

(IIIa)

In some embodiments the support may be a solid polymer.

In some embodiments the support may be a solid polymer selected from controlled-porosity glass and polystyrene.

In some embodiments the support may be polystyrene.

In some embodiments the support may be controlled-porosity glass.

In some embodiments the support may be functionalised with a primary amino group. This may form the reactive point of attachment to the support.

In some embodiments the support may be controlled-porosity glass functionalised with a primary amino group (for example Amino-SynBase™).

In some embodiments $PG^1$ may be selected from trityl, dimethoxytrityl and trimethoxytrityl.

In some embodiments $PG^2$ may be selected from acetyl, benzoyl, 2,2,2-trichloroethylcarbonyl, paramethoxybenzyl, methyl, tetrahydropyranyl, triethylsilyl, triisopropylsilyl, trimethylsilyl, tertbutyldimethylsilyl and methoxyethyl.

In some embodiments $PG^2$ may be acetyl. Where an immobilised molecule is base labile, this allows for an efficient synthesis in which removal of the $PG^2$ group and cleavage from the resin may be accomplished in a single step.

In some embodiments $PG^1$ may be dimethoxytrityl and $PG^2$ may be acetyl.

In some embodiments immobilisation of the compound of formula (II) in step i) may occur mainly at the 2'-hydroxy position.

When immobilisation occurs mainly at the 2'-hydroxy position, this may be >50%, >60%, >70%, >80%, >90% or 100% of the total immobilisation (i.e. the total covalent binding of both secondary hydroxyl groups to the support).

In some embodiments the tetraalkylammonium pyrophosphate may be tetrabutylammonium pyrophosphate.

In one embodiment there is provided a process for preparing a compound of formula (I) or a salt thereof comprising:

i. Providing a compound of formula (II) or a salt thereof:

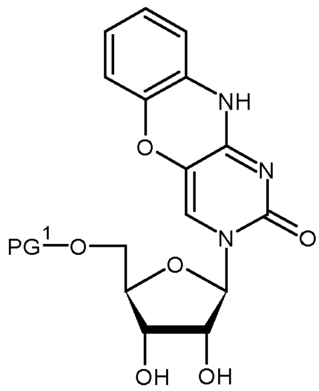

(II)

Where $PG^1$ is selected from trityl, dimethoxytrityl and trimethoxytrityl;

ii. Immobilising the compound of formula (II) or a salt thereof by linking one of its secondary alcohol groups to a controlled-porosity glass support;

iii. Capping any remaining secondary alcohol groups with a protecting group $PG^2$ selected from acetyl, benzoyl, 2,2,2-trichloroethylcarbonyl, paramethoxybenzyl, methyl, tetrahydropyranyl, triethylsilyl, triisopropylsilyl, trimethylsilyl, tertbutyldimethylsilyl and methoxyethyl;

iv. Removing the protecting group $PG^1$;

v. Reacting the exposed primary alcohol group with a compound of formula (III):

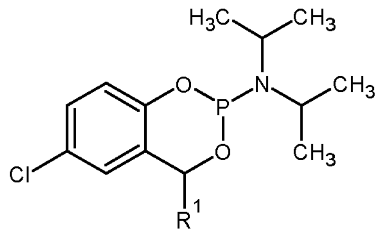

(III)

Where $R^1$ is a $C_{1-3}$alkyl group;

vi. Oxidising the resultant phosphorus (III) compound to a phosphorus (V) compound;

vii. Reacting the phosphorus (V) compound with tetrabutylammonium pyrophosphate to generate a triphosphate;

viii. Removing the protecting group $PG^2$; and

ix. Cleaving the resultant triphosphate from the support to generate a compound of formula (I) or salt thereof.

In one embodiment there is provided a process for preparing a compound of formula (I) or a salt thereof comprising:

i. Providing a compound of formula (II) or a salt thereof:

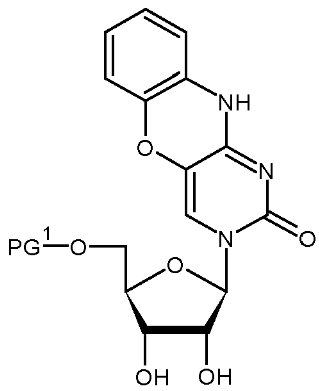

(II)

Where $PG^1$ is dimethoxytrityl;
ii. Immobilising the compound of formula (II) or a salt thereof by linking one of its secondary alcohol groups to a controlled-porosity glass support;
iii. Capping any remaining secondary alcohol groups with a protecting group $PG^2$ which is acetyl;
iv. Removing the protecting group $PG^1$;
v. Reacting the exposed primary alcohol group with a compound of formula (III):

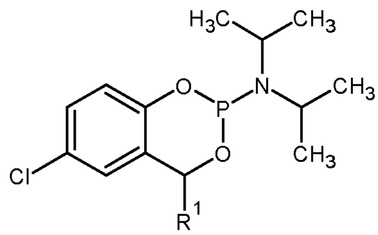

(III)

Where $R^1$ is a methyl group;
vi. Oxidising the resultant phosphorus (III) compound to a phosphorus (V) compound;
vii. Reacting the phosphorus (V) compound with tetrabutylammonium pyrophosphate to generate a triphosphate;
viii. Removing the protecting group $PG^2$; and
ix. Cleaving the resultant triphosphate from the support to generate a compound of formula (I) or salt thereof.

Suitable conditions and reagents to effect each of steps i) to ix) above are known to the skilled person or can be found in the Detailed Description.

In some embodiments immobilising the compound of formula (II) or salt thereof in step ii) may be accomplished by a coupling reagent (for example succinic anhydride catalysed by dimethylaminopyridine when the support is functionalised with a primary amino group).

In some embodiments reaction of the exposed primary alcohol group with a compound of formula (III) may be accomplished using an activator (for example BTT activator or Activator 42®).

In some embodiments the phosphorus (III) compound in step vi) may be oxidised to a phosphorus (V) compound using aqueous pyridine and iodine.

In some embodiments cleaving the triphosphate from the support may be accomplished using basic conditions (for example by treating with AMA). When there is a base-labile support and a base-labile protecting group is chosen for $PG^2$, using these conditions allows simultaneous deprotection and cleavage.

*Labelled RNA Synthesis*

In one embodiment there is provided a composition for preparing a $tC^O$ labelled RNA molecule comprising a compound of formula (I) and a natural ribonucleotide triphosphate.

A "natural ribonucleotide triphosphate" comprises the appropriate natural ribonucleoside with a triphosphate

group bonded to the 5' hydroxy position. It is equivalent to a natural ribonucleoside triphosphate. In some embodiments a natural ribonucleotide triphosphate may be selected from cytidine 5'-triphosphate, uridine 5'-triphosphate, adenosine 5'-triphosphate and guanidine 5'-triphosphate. A composition of natural ribonucleotide triphosphates (i.e. one comprising a ribonucleotide triphosphate as defined herein) may comprise combinations of varying amounts of these building blocks, in amounts sufficient to construct the target RNA molecule (for example as provided in NTP mix).

In one embodiment there is provided the use of a compound of formula (I) or a salt thereof to enzymatically prepare a $tC^O$ labelled RNA molecule.

A $tC^O$ labelled RNA molecule comprises at least one $tC^O$ residue but is otherwise similar to the natural RNA molecule (i.e. one with an unmodified cytosine residue at the same location as the $tC^O$ residue).

In some embodiments a $tC^O$ labelled RNA molecule may comprise >10%, >20%, >30%, >40%, >50%, >60%, >70%, >80%, >90% or 100% of $tC^O$ residues in place of unmodified cytosine residues.

In some embodiments a $tC^O$ labelled RNA molecule may comprise 10%-20%, 10%-30%, 10%-40%, 20%-50%, 30%-60%, 40%-70%, 50%-80% or 50%-90% of $tC^O$ residues in place of unmodified cytosine residues.

In one embodiment there is provided a process for preparing a $tC^O$ labelled RNA molecule comprising providing a DNA template to composition comprising a compound of formula (I) and a natural ribonucleotide triphosphate (for example a combination of varying amounts of cytidine 5'-triphosphate, uridine 5'-triphosphate, adenosine 5'-triphosphate and/or guanidine 5'-triphosphate in amounts sufficient to construct the target RNA molecule, for example as provided in NTP mix), then treating the resultant mixture with an RNA polymerase.

In some embodiments the $tC^O$ labelled RNA molecule may be a $tC^O$ labelled mRNA.

In some embodiments the $tC^O$ labelled RNA molecule may encode for a protein fused to a fluorescent protein. Example fusable fluorescent proteins include Green Fluorescent Protein (GFP) and mFruit family proteins. When the target protein is fluorescent (either inherently or due to a tag), it is possible to simultaneously visualise both the labelled RNA molecule and the protein it is being used to synthesise, giving a greater degree of mechanistic insight.

In some embodiments the $tC^O$ labelled RNA molecule may encode for a protein selected from H2B, calmodulin, H2B:GFP and calmodulin-3:GFP.

The terminology ":GFP" means that the protein target preceding the colon is fused to a Green Fluorescent Protein (GFP) family protein.

In some embodiments the $tC^O$ labelled RNA molecule may encode for H2B:GFP.

In some embodiments the $tC^O$ labelled RNA molecule may encode for calmodulin-3.

In some embodiments the RNA polymerase may be selected from T7 polymerase and SP6 polymerase.

In some embodiments a process for preparing a $tC^O$ labelled RNA molecule may be carried out in the presence of transcription buffer (e.g. 5X transcription buffer), magnesium salt (e.g. magnesium(II) chloride) and/or an RNase inhibitor (e.g. Ribolock).

In some embodiments a process for preparing a $tC^O$ labelled RNA molecule may be carried out in the presence of transcription buffer (e.g. 5X transcription buffer), magnesium salt (e.g. magnesium(II) chloride) and an RNase inhibitor (e.g. Ribolock).In some embodiments a process for preparing a process for preparing a $tC^O$ labelled RNA molecule may be carried out substantially as described in the experimental section (e.g. as detailed in the section headed "H2B:GFP RNA transcription and purification").

In one embodiment there is provided a kit for preparing a $tC^O$ labelled RNA molecule comprising:

i. A compound of formula (I);
ii. A composition of natural ribonucleotide triphosphates;
iii. An RNA polymerase; optionally
iv. A DNA template; and optionally
v. Instructions for use.

*Labelled RNA Translation*

In one embodiment there is provided the use of a $tC^O$ labelled mRNA molecule to prepare a protein encoded by the mRNA by translation.

"Translation" refers to the central biological process whereby mRNA is decoded in a ribosome to produce a specific polypeptide, which may fold into an active protein before performing its functions in a cell.

In one embodiment there is provided the use of a $tC^O$ labelled mRNA molecule to prepare a protein encoded by the mRNA by in vitro translation (for example, substantially as described in the part of the experimental section (e.g. as detailed under the heading "cell-free translation").

In some embodiments, in vitro translation may be performed using E.coli bacterial lysates and/or the Expressway® mini cell-free expression system.

In one embodiment there is provided the use of a $tC^O$ labelled mRNA molecule to prepare a protein encoded

by the mRNA by in cell translation (for example, substantially as described in the parts of the experimental section (e.g. as detailed under the headings "cell culture" and "electroporation or chemical transfection").

In some embodiments, in cell translation may be performed in human neuroblastoma cells (e.g. SH-SY5Y cells).

In one embodiment there is provided the translation of a $tC^{O}$ labelled RNA molecule into a protein.

In one embodiment there is provided the in vitro translation of a $tC^{O}$ labelled RNA molecule into a protein.

In one embodiment there is provided the in cell translation of a $tC^{O}$ labelled RNA molecule into a protein.

In some embodiments the encoded protein may be fused to a fluorescent protein (for example a GFP or mFruit family protein). When this is the case, it is possible to simultaneously visualise both the labelled RNA molecule and the protein it is being used to synthesise, giving a greater degree of mechanistic insight.

In some embodiments the $tC^{O}$ labelled mRNA and the encoded protein may be simultaneously analysed spatiotemporally using confocal microscopy (for example fluorescence confocal microscopy). This is a convenient way to simultaneously monitor a labelled RNA and protein, and RNA containing a fluorescent base analogue has never before been used in such live cell visualisation.

FIGURES

Figure 1: Schematic showing minimally perturbing $tC^{O}$ labelled RNA compared to a common externally labelled RNA

Figure 2: Basic synthetic scheme for the preparation of compound (I).

Figure 3: Incorporation of $tC^{O}$ into full length mRNA by T7 RNA polymerase assisted in vitro transcription. Denaturing agarose bleach gels showing RNA transcripts formed at five different $tC^{O}$TP/CTP ratios (0-100%). Direct visualization of $tC^{O}$ fluorescence (a) and after ethidium bromide staining (b). RNA samples were heat-denatured (65 °C for 5 min, 1.5 % bleach in the gel) prior to loading. (c) Same RNA transcripts upon harsher denaturation (70 °C for 10 min., 2 % bleach in the gel). The RiboRuler High Range RNA ladder was used.

Figure 4: Incorporation of $tC^{O}$ into full length mRNA by SP6 and T7 RNA polymerase assisted in vitro transcription, (a) Denaturing agarose bleach gels showing RNA transcripts formed at five different $tC^{O}$TP/CTP ratios (0-100%). The produced RNA was visualized directly by $tC^{O}$ fluorescence (left image) and after ethidium bromide staining (right image). The RNA samples were heat-denatured (65 °C for 5 min, 1.5 % bleach in the gel) prior to loading on the gel. (b) Denaturing bleach gels of the same RNA transcripts as in (A) but at stronger denaturing conditions (70 °C for 10 min., 2 % bleach in the gel). The RiboRuler High Range RNA ladder was used.

Figure 5: Spectroscopic characterization of in vitro synthesized $tC^{O}$-modified RNA transcripts. Four reactions charged with different molar fractions of $TC^{O}$TP in the total cytosine triphosphate pool ($TC^{O}$TP + CTP) were performed. The product transcripts were purified to wash out unreacted triphosphates prior to characterization. All reactions were performed as independent duplicates and the results are presented as mean $\pm$ standard deviation. a) UV-vis absorption spectra normalized to A = 1 at the RNA band, *ca*. 260 nm with increased $tC^{O}$-absorption centred at 360 nm growing in with an increasing $tC^{O}$ to C ratio. Inset: $TC^{O}$TP absorption normalized to A = 1 at the $tC^{O}$-band $\lambda$max (360 nm). b) Plain bars: Fraction of incorporated $tC^{O}$ (relative to the total amount of incorporated cytosines, i.e. $tC^{O}$ + C) in the transcripts. Checkered bars: Ratio of first-order reaction rate constants for CTP vs. $TC^{O}$TP consumption. c) Solid lines: UV-vis absorption spectra (normalized to A = 1 at the RNA band, *ca.* 260 nm) showing the $tC^{O}$-band centred at 368-369 nm. Dashed lines: Emission spectra normalized to I = 1 at $\lambda$max (457 nm and 459 nm for the 25% and 100% transcript, respectively). For clarity, the emission spectra for the 50% and 75% reactions were omitted. d) Plain bars: Fluorescence quantum yields. Striped bars: Fluorescence lifetime.

Figure 6: Cell-free translation of calmodulin-3. a) Coomassie staining and b) Western Blot (WB) of the in-vitro translation reactions. NTC: no template control; +: kit template DNA control. The PageRuler Prestained Protein Ladder was used. c) Quantification by WB and densitometry analysis (mean of 4 replicates).

Figure 7: Translation efficiency of modified RNA constructs in human cells and validation of $tC^{O}$ as an intracellular tracking probe. The H2B:GFP encoded protein was observed by confocal microscopy and quantified by flow cytometry for each $tC^{O}$-incorporated RNA constructs. Representative images (3x zoomed-in, scale bars: 10 $\mu$m), scatter plots and histograms, show the signal distribution in single living cells at (a, b) 24 h post-electroporation or (c) 48 h post-chemical transfection. The boxplots display the GFP mean fluorescence intensities (MFI GFP) up to 72 h from 3 independent experiments performed in triplicate. (d) Cells overexpressing mRFP-Rab5 (early endosome biomarker) were transfected with 75 % $tC^{O}$ mRNA and followed overtime to validate $tC^{O}$ as an intracellular tracking probe (white arrows) not altering the translation, scale bars: 10 $\mu$m. (e) Cells were analysed 24 h post-electroporation or post-transfection with non-labelled (NL) or Cyanine5-labelled (Cy5) eGFP encoding mRNAs (TriLink®), scale bars: 10 $\mu$m. (f) The impact of $tC^{O}$ or Cy5 incorporation on RNA translation was expressed as the ratio of MFI GFP relative to the non-labelled RNA for all constructs.

Figure 8: Translation efficiency of the modified RNA constructs in human cells and cytotoxicity assessment. Representative confocal images (large view, scale bar: 10 μm) of RNA-tC$^{O}$ constructs and mRNAs from TriLink® transfected by (a, e) electroporation or (b, f) chemical transfection. (c) Percentages of positive cells for H2B:GFP at 24 h, 48 h and 72 h post-transfection with RNA-tC$^{O}$ constructs. (d) Representative histogram of the GFP signal distribution in single living cells at 48 h post-chemical transfection. Cytotoxicity assessment performed 24 h (g) post-electroporation or (h) post-chemical transfection using the LDH cell membrane integrity assay.

## DETAILED DESCRIPTION

*Example 1: Synthesis of Modified Nucleobase Triphosphates*

Compound (I) may be prepared according to the scheme shown in Fig. 2. Unless otherwise noted reagents were commercially available and used without further purification. The following reagents used for the triphosphorylation were bought from Sigma-Aldrich: DCA deblock for ÄKTA, CAP A for ÄKTA, CAP B1 and B2 for ÄKTA, BTT Activator. $^{1}$H (500 MHz) and $^{13}$C (126 MHz) NMR spectra were recorded at 300 K on a Bruker 500 MHz system equipped with a CryoProbe. $^{31}$P (202 MHz) NMR spectra were recorded at 300 K on a Bruker 500 MHz system. All shifts are recorded in ppm relative to the deuterated solvent (DMSO-d6, $CDCl_3$ or $D_2O$).

**3-((2R,3R,4S,5R)-5-((bis(4-methoxyphenyl)(phenyl)methoxy)methyl)-3,4-dihydroxytetrahydrofuran-2-yl)-3*H*-benzo[*b*]pyrimido[4,5-*e*][1,4]oxazin-2(10*H*)-one 1**

Compound 1 was prepared according to the literature (Füchtbauer, A. F. *et al., Sci . Rep. 7,* 2393 [**2017**])

MS (ESI-) [M-H]- = 634.5. $^{1}$H NMR (500 MHz, DMSO-d6) δ 10.61 (bs, 1H), 7.42 (d, J = 7.7 Hz, 2H), 7.27 - 7.35 (m, 7H), 7.22 (t, J = 7.1 Hz, 1H), 6.90 (dd, J = 8.6, 4.2 Hz, 4H), 6.75 - 6.87 (m, 3H), 6.46 (d, J = 7.8 Hz, 1H), 5.71 (d, J = 3.6 Hz, 1H), 5.49 (bs, 1H), 5.18 (bs, 1H), 4.08 (d, J = 5.3 Hz, 1H), 4.04 (s, 1H), 3.94 (s, 1H), 3.71 (s, 3H), 3.70 (s, 3H), 3.29 (d, J = 4.8 Hz, 1H), 3.16 (d, J = 9.1 Hz, 1H).

**CPG solid support 3**

Amino-SynBase™ CPG 500/110 (LCAA) from LinkTech (Nu. 1397-C025, 1 g, 0.08 mmol) was activated by shaking in trichloroacetic acid 3% in DCE (8 mL, 0.08 mmol) for 18 h. The activated support was then filtered off and washed with 9:1 triethylamine:diisopropylethylamine (20 mL), dichloromethane (20 mL) and diethyl ether (20 mL). The activated support was dried under vacuum for 2 days before use. Subsequently, the support (1 g, 0.08 mmol), succinic anhydride (0.345 g, 3.44 mmol) and N,N-dimethylpyridin-4-amine (0.070 g, 0.57 mmol) were suspended in dry Pyridine (3 mL) under $N^2$. The reaction mixture was then gently shaken at RT for 4 h. After 4 h, solvent was filtered off and the support washed successively with pyridine (20 mL), dichloromethane (20 mL), diethyl ether (20 mL) and air-dried. Negative ninhydrin test on a small portion of support proved full succinylation. Succinylated CPG could thereafter be kept at room temperature for several months.

**CPG-supported (2R,3R,4R,5R)-5-((bis(4-methoxyphenyl)(phenyl)methoxy)methyl)-4-hydroxy-2-(2-oxo-2,10-dihydro-3*H*-benzo[*b*]pyrimido[4,5-*e*][1,4]oxazin-3-yl)tetrahydrofuran-3-yl acetate 4**

In a 10 mL syringe with PTFE filter, succinylated support 3 (1.420 g, 82 μmol/g, 0.12 mmol), DMAP (0.028 g, 0.23 mmol), DIC (719 μl, 4.64 mmol), 1 (0.076 g, 0.12 mmol) and triethylamine (49 μl, 0.35 mmol) were suspended pyridine (5 mL). The mixture was gently shaken for 18 h at RT. After 18 h, the syringe was purged and the support washed with pyridine (5 mL), dichloromethane (5 mL) and diethyl ether. Subsequently, in the same syringe, DMAP (0.028 g, 0.23 mmol), diisopopylcarbodiimide (719 μl, 4.64 mmol), triethylamine (49 μl, 0.35 mmol) and 2,3,4,5,6-pentachlorophenol (0.309 g, 1.16 mmol) were added to the support and suspended in pyridine (4 mL). The mixture was gently shaken for 4 h at RT before a solution of piperidine (2 mL, 20% in DMF - for capping of the unreacted carboxylic acids on the support) was added for 1 min (longer exposure time will reduce loading as piperidine cleaves the ester bonds with the nucleoside), then quickly washed away with DMF (3x5 mL), dichloromethane (5 mL) and diethyl ether (5 mL). Finally, the resin was shaken in a CAP A + CAP B mix (50/50 v/v) for 2 hours under argon atmosphere, then washed with DMF (5 mL), dichloromethane (5 mL), diethyl ether (5 mL) and argon-dried (final loading: 13 μmol/g - determined by reading optical density of a DMT solution cleaved from a weighed amount of support - E=70000 M-1.cm-1 at 498 nm). Final loading can be increased by performing a second coupling with 1 in the same conditions before capping (typical loading after second coupling 20-25 μmol/g). Concentrating the reaction mixture and washing the residue multiple times with water and diethyl ether allows recovery of nearly 85% of unreacted nucleoside 1.

**6-chloro-*N,N*-diisopropyl-4*H*-benzo[*d*][1,3,2]dioxaphosphinin-2-amine** 5

Compound 5 was prepared according to the literature (Ducho, C. et al., J . Med . Chem. 50, 1335-1346 [2007]). Briefly, 5-chlorosalicylic acid was reduced with LAH (0.5 equiv.) at -20 °C and the resulting 5-chlorosalicylic alcohol was cyclized into 2,6-dichloro-4H-benzo[d][1,3,2]dioxaphosphinine using $PCl_3$ (1.2 equiv.) and triethylamine (2.3 equiv.) at -20 °C under argon. Low temperature and use of triethylamine as the base were decisive in avoiding rapid and quantitative Arbuzov rearrangement of the desired product into the more stable 2,5-dichloro-3H-benzo[d][1,2]oxaphosphole 2-oxide. The crude 2,6-dichloro-4H-benzo[d][1,3,2]dioxaphosphinine was subsequently treated with diisopropylamine (3 equiv.) for 2 h at room temperature. The mixture was then filtered under argon, concentrated to dryness and taken in 20 % diisopropylamine in heptane. Quick filtration on a small silica gel plug allowed desired compound 5 as a colourless oil, crystallizing over time at -20 °C. Any attempt of more thorough column chromatography on compound 5 would lead to quantitative Arbuzov rearrangement.

$^1$H NMR (500 MHz, DMSO-d6) δ = 7.23 (dd, J = 8.6, 2.6 Hz, 1H), 7.20 (d, J = 2.4 Hz, 1H), 6.92 (d, J = 8.6 Hz, 1H), 5.06 (dd, J = 14.7, 5.2 Hz, 1H), 4.89 (dd, J = 19.6, 14.8 Hz, 1H), 3.53 - 3.63 (m, 2H), 1.15 - 1.19 (dd, J = 8.0, 7.0 Hz, 12H).$^{31}$P NMR (202 MHz, DMSO-d6) δ = 136.00 (s, 1P).

***Bis*(tetrabutylammonium) dihydrogen diphosphate 6**

Compound 6 was prepared according to the literature (Warnecke, S. & Meier, C., J. Org. Chem. 74, 3024-3030 [2009]).

$^1$H NMR (500 MHz, $D_2O$) δ 3.04 - 3.13 (m, 16H), 1.53 (bs, 16H), 1.24 (h, J=7.3, 16H), 0.83 (t, J=7.4, 24H). $^{31}$P NMR (202 MHz, $D_2O$) δ = -10.78 (s, 2P).

**((2R,3S,4R,5R)-3,4-dihydroxy-5-(2-oxo-2,10-dihydro-3*H*-benzo[*b*]pyrimido[4,5-*e*][1,4]oxazin-3-yl)tetrahydrofuran-2-yl)methyl triphosphate 7**

Reactions were performed in a 5 mL syringe with PTFE filter loaded with 4 (800 mg, 0.016 mmol) under an argon atmosphere and with shaking.

Steps were performed as following:

a. *5'-DMT removal:* the support was washed with a flow of DCA deblock until the filtrate was colourless, then washed with ACN (5 × 5 mL).

b. *Coupling:* N,N-diisopropyl-4H-benzo[d][1,3,2]dioxaphosphinin-2-amine 5 (345 mg, 1.36 mmol) was dissolved in 4.8 mL ACN and reacted portion wise with the support (3 equal couplings with reaction times 60 s - 60 s - 90 s respectively). To each coupling, an activator (e.g. BTT activator (2.4 mL) or Activator 42) was also added. The support was subsequently washed with ACN (3x5 mL).

c. *Oxidation:* Pyridine/Water/Iodine (9/1/12.7 v/v/w, 5mL) for 45 s, followed by ACN wash (3x5 mL) and drying of the support in an argon flow.

d. *Triphosphorylation:* Two injections of bis(tetrabutylammonium) dihydrogen diphosphate 6 (0.5 M, 5 ml) for 15 min and 18 hours, respectively. The support was subsequently rinsed with DMF (5 mL), water (3x5 mL), ACN (5 mL) and then dried in an argon flow.

e. *Cleavage and Purification:* Cleavage of the triphosphate was done in 2 h at room temperature with AMA (50/50 v/v mix of 23 % aq. $NH_4OH$ and 40 % aq. methylamine, 5 mL). After 2 hours, the AMA filtrate was purged in a round-bottom flask and the support was rinsed 3 times with 23% aq. $NH_4OH$ solution. After freeze-drying of the mixture, purification by HPLC (Waters Acquity HSS T3 column, 2.1x50 mm, 0.4 mL/min, 2 to 99 % 50 mM $NH_4OAc$ in water 80:20 EtOH) was performed to allow compound 7 (5.6 mg, 62.0 % determined from UV absorbance) as a light-yellow solid (ammonium salt). The same level of purity could be achieved with ionexchange HPLC using a semi-preparative Dionex DNAPac PA100 column (9 × 250 mm) on an ÄKTA pure 25 HPLC system using a gradient from water to 20 % 1M $NH_4HCO_4$ (pH 7.8) in 30 min at a flow rate of 4 mL/min.

HRMS (ESI-TOF) m/z calc. for $C_{15}H_{18}N_3O_{15}P_3$ [M+H]+: 574.0029, found : 574.0013; m/z calc. for C15H18N3O15P3 [M-H]-:571.9878, found: 571.9872. $^1$H NMR (500 MHz, D2O) δ 7.44 (s, 1H), 6.84 - 6.94 (m, 3H), 6.79 (dd, J = 7.5, 1.7 Hz, 1H), 5.91 (d, J = 4.9 Hz, 1H), 4.36 (t, J = 4.8 Hz, 1H), 4.29 (t, J = 5.1 Hz, 1H), 4.25 (d, J = 4.1 Hz, 3H). $^{13}$C NMR (126 MHz, D2O) δ 155.8, 154.8, 142.4, 129.4, 124.9, 124.3, 122.3, 116.6, 88.8, 82.8, 73.4, 69.7, 64.5. $^{31}$P NMR (202 MHz, $D_2O$) δ -10.89 (d, J = 18.5 Hz, 1P), -11.46 (d, J = 19.7 Hz, 1P), -23.21 (t, J = 19 Hz, 1P).

Compound (I) can also be made by a slightly modified route wherein the coupling step (b above) is carried out with a modified phosphoramidite such as 6-chloro-N,N-diisopropyl-4-methyl-4H-benzo[d][1,3,2]dioxaphosphinin-2-amine 8 (compound (IIIa) above). This reagent has been found to be more easily prepared, and compound 8 is obtainable

in a yield of 60% compared to around 3-10% for the preparation of compound 5 under the conditions in this specification.

**6-chloro-*N,N*-diisopropyl-4-methyl-4*H*-benzo[*d*][1,3,2]dioxaphosphinin-2-amine 8**

5-chloro-2-hydroxybenzaldehyde was reacted with methylmagnesium bromide (2.5 equiv.) at -20 °C and the resulting 4-chloro-2-(1-hydroxyethyl)phenol was cyclized into 2,6-dichloro-4-methyl-4H-benzo[d][1,3,2]dioxaphosphinine using $PCl_3$ (1.2 equiv.) and triethylamine (2.3 equiv.) at -20 °C under argon. The crude 2,6-dichloro-4-methyl-4H-benzo[d][1,3,2]dioxaphosphinine was subsequently treated with diisopropylamine (3 equiv.) for 2 h at room temperature. The mixture was then filtered under argon, concentrated to dryness and taken in 20 % diisopropylamine in heptane. Quick filtration on a small silica gel plug furnished desired compound 8 as a colourless oil.

$^1$H NMR (500 MHz, DMSO-d6) $\delta$ = 6.96 (d, J = 8.5 Hz, 1H), 6.87 (d, J = 8.5 Hz, 1H), 6.74 (d, J = 8.4 Hz, 1H), 5.19 - 5.26 (m, 1H), 5.16 (dq, J = 10.4, 6.6 Hz, 1H), 3.57 (tdt, J = 13.6, 10.6, 6.8 Hz, 2H), 1.63 (d, J = 6.6 Hz, 3H), 1.55 (d, J = 6.4 Hz, 2H), 1.16 - 1.19 (m, 24H). $^{31}$P NMR (202 MHz, DMSO-d6) $\delta$ = 137.63 (s, 1P), 127.90 (s, 1P).

*Example 2: Cell-free in vitro transcription*

The utility of compound (I) in RNA labelling was demonstrated by its cell-free in vitro transcription to produce fluorescent full-length messenger RNA (mRNA), from a DNA template encoding for H2B histone protein fused to GFP (H2B:GFP).

The template was codon optimized to limit the number of C repeats, preventing self-quenching and improving brightness. Efficient transcription and $tC^O$ incorporation was observed using two different bacteriophage RNA polymerases, T7 and SP6 at $tC^O$TP/canonical CTP ratios ranging from 0 to 100% (full replacement), as demonstrated by agarose bleach gel electrophoresis (Fig. 3a for T7 and Fig. 4a for SP6).

All RNA transcripts run as one single band on the gels, with a size corresponding to the expected 1247 nt mRNA product (H2B:GFP), demonstrating that the full-length mRNA is formed. The $tC^O$-containing mRNA bands could be directly visualized upon 302 nm excitation (Fig. 4a); the increasing band intensities with increasing $tC^O$TP/CTP reaction ratio supported successful concentration-dependent incorporation of tC°. Re-visualization of the gel after ethidium bromide staining (Fig. 3b ) provided a further qualitative indication that $tC^O$ incorporation does not reduce the reaction yield.

Furthermore, no shorter transcripts were observed, suggesting that T7 processes $tC^O$TP correctly and without premature abortion. Higher order bands are apparent in all lanes of the gel (Fig. 3b and 4b) but were removed upon heat denaturation, suggesting the presence of RNA secondary structures. Notably, this feature appears independently of the CTP/$tC^O$TP-ratio, indicating that the effect is not specific to the modified cytosine base.

Therefore, these results demonstrate that $tC^O$ can be successfully incorporated into full-length RNA transcripts even under conditions where all canonical CTP is replaced with $TC^O$TP (0% CTP; i.e. 100% C-labelling efficiency).

*Example 3: Spectroscopic characterization of in vitro synthesized $tC^O$-modified RNA transcripts*

A spectroscopic approach was used to quantify the incorporation efficiency of $tC^O$TP, compared to the canonical CTP. To enable this, all RNA transcripts were purified using a Monarch RNA Cleanup kit, ensuring complete removal of unreacted $tC^O$TP. Absorption spectra (Fig. 5a) showed the appearance of a band centred at *ca*. 370 nm in samples with the incorporated cytosine analogue $tC^O$, consistent with the spectral profile of this fluorescent base analogue (Fig. 5a).

By relating the absorption of the purified RNA transcripts at 260 nm, which reflects their total concentration, to the absorption at 370 nm (emanating exclusively from $tC^O$), the relative rate constants for the incorporation of CTP and $TC^O$TP (kC and $ktC^O$, respectively, see later for details). The calculated quotients kC/$ktC^O$ (Fig. 5b) have values close to or slightly above unity (0.96-1.4, Fig. 5b), demonstrating that the T7 polymerase displays no substantial preference for the canonical CTP over $tC^O$TP in the *in vitro* reactions. This supports that the tricyclic chemical modification of cytosine is indeed minimally perturbing in the transcription process.

The emissive behaviour of $tC^O$ was also investigated in the mRNA transcripts exploring the relation to the $tC^O$TP fraction added to the initial reaction mixture. A substantial decrease in fluorescence quantum yield (from 0.18 to 0.09, Fig. 5d) was observed with increasing $tC^O$ incorporation. This was accompanied by a decrease in fluorescence lifetime (from 4.3 ns to 3.2 ns) and a slight redshift of the emission spectrum (*ca.* 4 nm, Fig. 5c).

This may be ascribed to electronic interaction (coupling) of molecular states of the $tC^O$ fluorophore and self-quenching effect caused by the expected increasing concentration of vicinal $tC^O$s. Importantly, this quenching effect at high $tC^O$ fractions is balanced by the large overall number of incorporations and does not prevent visualization of the mRNA, even for transcripts where all Cs are replaced by $tC^O$s.

*Example 4: Translation of* $tC^O$-labelled *mRNA in bacterial lysates*

In order to verify the functionality of the $tC^O$-labelled mRNA transcripts the translation of a $tC^O$-labelled Calmodulin-3 mRNA in cell-free conditions using bacterial lysates was investigated. The labelled mRNAs encoding for the 17 kDa protein were transcribed from a commercial Calmodulin-3 DNA template plasmid using the same $tC^O$TP/CTP ratios as for the H2B:GFP encoding mRNA (0 to 100% of $tC^O$TP). After RNA purification and cell-free translation, the presence of Calmodulin-3 was confirmed by Coomassie staining (Fig. 6a) as well as Western Blot (Fig. 6b). Satisfactorily we observed stable expression levels when increasing the $tC^O$ content of the transcripts, ranging from 80%-137% of that obtained using a commercially available, unlabelled DNA template control (Figure 6c).

*Example 5: Translation efficiency of* $tC^O$-labelled *mRNA in human cells*

Electroporation was used to introduce in vitro-transcribed $tC^O$-labelled mRNA transcripts into human neuroblastoma SH-SY5Y cells. Taking advantage of them encoding for a fluorescent fusion protein with nuclear localization (H2B:GFP), the translation was detected by fluorescence (Figs. 7 and 8). To improve stability and reduce cytosolic degradation, the mRNAs were capped with a 5'-Cap 0 analogue and 3'-protected by poly-adenylation (by *ca*. 300 nt).

Live-cell confocal microscopy and flow cytometry showed that GFP fluorescence in the cell nuclei could be detected in 32, 25, 18, and 12% of the cells 24 hours post-electroporation for mRNA's containing 25, 50, 75 and 100% of $tC^O$, respectively (Fig. 7a and Fig. 8a). In comparison, the transfection efficiency with unmodified mRNA was 46%. This provides the first observation that a fluorescent base analogue-modified RNA transcript can be accurately and efficiently translated by human ribosomal machineries, resulting in a correctly localized and folded protein product.

Using flow cytometry, the levels of H2B:GFP fluorescence in the cells was quantified (Fig. 7b), showing a decrease in mean cellular H2B:GFP fluorescence intensity upon increasing the percentage of $tC^O$ in the transcript (approximately one order of magnitude difference between 0% and 100% of $tC^O$ (Fig. 7a and 7f). This suggests that under these conditions, translation, as opposed to transcription, is somewhat impeded by the $tC^O$ modification, especially at the highest incorporation fraction.

Importantly, no evidence was found of mRNA-induced cell toxicity at 24 hours post-electroporation (Fig. 8g). Of significant note is that the mean fluorescence intensity of translated protein in cell cultures electroporated with a commercial enhanced GFP-encoding mRNA tagged with Cy5 *via* conjugation to UTPs (*ca.* 25% of all U positions of this mRNA are labelled) was only 17% of that in cell cultures electroporated with the corresponding non-labelled sequence (Fig. 7f). This is comparable to the effect of a 50%-75% labelled $tC^O$ transcript, which suggests that Cy5 modifications affect the ribosome translation capability more than $tC^O$ does.

It is evident from the images in Fig. 7a and 7e that neither the fluorescent $tC^O$-labelled mRNA nor the Cy5-labelled mRNA can be detected inside cells after electroporation due to their low/diffused cytoplasmic concentration. The delivery of the H2B:GFP transcripts was therefore probed using a chemical transfection reagent (lipofectamine), which is also more relevant from a drug delivery perspective. This resulted in the successful production of H2B:GFP (Figs. 7c, 8b and 8d) irrespective of the $tC^O$ content, but with albeit lower delivery efficiencies (3.8-4.6 % of H2B:GFP-positive cells for $tC^O$-labelled transcripts vs. 12-32% post-electroporation). This reflects the relatively poor transfectability of SH-SY5Y cells compared to many other cell lines rather than the transfectability of $tC^O$-labelled constructs, as further supported by the finding that cultures transfected with non-modified mRNA displayed a virtually identical response (4.5 % of cells expressing H2B:GFP).

H2B:GFP fluorescence was found to increase gradually with time between 24 h and 72 h (Fig. 7c), which is a contrasting behaviour compared to electroporated cells (Fig. 7a), suggesting that the lipofectamine-mRNA complexes are continuously internalized and, potentially, that endocytosed complexes progressively release more transcripts with time, counteracting the degradative effect in the cytosol.

When delivered using lipofectamine, the $tC^O$-labelled mRNAs were found to promote very similar H2B:GFP translation compared to the corresponding non-labelled mRNA, as indicated by the fluorescence levels in Fig. 7f. The Cy5-tagged mRNA, on the other hand, results in an average fluorescence level that is 80 % lower than that of its corresponding non-labelled transcript. This suggests that $tC^O$ does not impair the ability of mRNA to be processed by ribosomes upon chemical transfection, possibly because of an absence of charge and reduced steric hindrance, whereas Cy5, currently the most common commercial fluorophore for mRNA labelling, quite considerably impacts the translation process and/or interferes with a native-like uptake process of mRNA since it introduces significant amphiphilicity to the mRNA and, hence, possibly non-native interactions between the CY5-mRNA and the lipophilic membrane constituents.

Importantly, the complexation of the $tC^O$-labelled mRNA with lipofectamine enabled its direct visualization inside cells using live cell confocal microscopy (Fig. 7c). This represents the first observation of fluorescent base analogue-labelled nucleic acids inside live cells. It was also found to be possible to simultaneously visualize, in real time, the uptake and subsequent translation of a fluorescent base analogue-modified mRNA by time-lapse recordings. We observed co-localization of the $tC^O$ signal with an mRFP-labelled Rab5 protein, thus highlighting that the mRNA transits

through the early endosome (Fig. 7d). Consequently, this technology allows tracking of both the intrinsically labelled mRNA transcripts and their translation products live, to gather spatiotemporal information on the translation product, even with as low as 50 % $tC^O$ content. This demonstrates the flexibility and versatility of this new labelling approach where fine-tuning of $tC^O$ content can be utilized to optimize the mRNA for specific drug delivery applications.

*Example 6: Biochemical Methods*

**Generation of H2B:GFP DNA template**

The original coding sequence for H2B:GFP was taken from pCS2-H2B:GFP plasmid (Addgene, Plasmid #53744, manually codon-optimized to minimize the occurrence of poly-Cn stretches (n<3), in silico-assembled with an additional T7 promoter and other desired features (Shine-Dalgarno/Kozak consensus sequences for enhancement of translation and a 3xStop, respectively at the 5' and 3' of the coding sequence itself, plus the needed HindIII/SnaBI restriction sites, to generate the ligation-prone sticky ends) and ordered from Twist Bioscience as a synthetic gene block. The obtained sequence was then PCR-amplified, using a Phusion Hot Start High-Fidelity Taq (Thermo Scientific), and subcloned into a HindIII/SnaBI-digested (Fast Digest enzymes, Thermo Scientific) empty pCS2 backbone. After ligation with T4 ligase for 1 h at room temperature (Roche), DH5α E. coli competent cells (Invitrogen) were transformed following the recommended protocol, and obtained colonies were screened by colony-PCR. The selected colony was then inoculated into a midiprep-scale volume of liquid Luria-Bertani growth medium (VWR) and plasmid DNA isolated using a PureLink Fast Low-Endotoxin Midi Plasmid Purification Kit (Thermo Scientific). The purified plasmid was finally digested again with HindIII/SnaBI and gel-purified, to generate the transcription template with the desired size.

Primers (Eurofins Genomics):

Twist-H2B.F: GAAGTGCCATTCCGCCTGAC

Twist-H2B.R: CACTGAGCCTCCACCTAGCC

**H2B:GFP RNA transcription and purification**

*In-vitro* transcription reactions, for T7 and SP6 polymerases (Thermo Scientific), were assembled as recommended by the corresponding protocols, with a few modifications that resulted in a consistently increased yield in all conditions:

1. 5X Transcription buffer - 10 μl
2. NTP Mix, 10 mM each (2 mM final concentration) - volume depending on batch for $tC^OTP$
3. Linearized template DNA 1 μg - volume depending on concentration
4. RiboLock RNase Inhibitor - 1.25 μl (50 U)
5. T7/T3/SP6 RNA Polymerase - 3 μl (60 U, double compared to recommendations)
6. $MgCl_2$ 4 mM final concentration (increased as recommended by Thomen, P. et al. Biophys. J. 95, 2423-2433 [2008])
7. DEPC-treated Water qsp 50 μl

*In-vitro* transcriptions were always performed at 20 °C for 14 h, then RNAs were purified using a Monarch RNA Cleanup kit (NEB), or homemade equivalent buffers and regenerated columns following the same rationale. It was possible to partially recover unreacted $TC^OTP$ from the transcription mixtures by HPLC to re-use for further assays. For cellular studies, each batch of RNA was then enzymatically added with a polyA tail (with a Poly(A) Polymerase, NEB protocol #M0276 with incubation extended to 1 hour) and a Cap 0 analogue (using a Vaccinia capping system, NEB protocol #M2080), following the recommended procedures.

**Denaturing bleach-agarose gels**

For a qualitative check of all in vitro synthesized RNAs, a denaturing agarose gel was run, in presence of 1,5 % bleach (Sigma Aldrich), as recommended in Aranda, P. S., LaJoie, D. M. & Jorcyk, C. L., Electrophoresis 33, 366-369 [2012]. RNAs were first mixed with a 6x DNA loading dye (Invitrogen) and then heat-denatured at 70°C for 10 min in a heating block, then immediately transferred and kept on ice. The RiboRuler High Range RNA Ladder (Thermo Scientific) underwent the same treatment; 2 μl of RNA ladder were loaded along the samples and the gel was run at constant voltage (70 V) for 1 h and then imaged, under UV transillumination (302 nm) using a ChemiDoc Touch (BioRad). To counterstain the whole gel, and especially the lanes without $tC^OTP$-containing samples, a standard ethidium bromide staining was finally performed at room temperature for 10 min and gentle rocking, followed by two washes in TAE and

then a final wash in distilled water (10 min each).

**Cell culture**

Human neuroblastoma SH-SY5Y cells (Sigma-Aldrich) were grown in a 1:1 mixture of minimal essential medium (HyClone) and nutrient mixture F-12 Ham (Sigma-Aldrich) supplemented with 10% fetal bovine serum (FBS), 1% non-essential amino acids (Lonza) and 2 mM L-glutamine. For the tracking experiments, an in-house generated model of human hepatic Huh-7 cells stably overexpressing mRFP-Rab5 were cultured in DMEM/GlutaMax/High glucose (Gibco) supplemented with 10 % FBS. The cells are detached with trypsin-EDTA 0.05 % (Gibco) and passaged twice a week.

**Electroporation or chemical transfection**

Cells were electroporated either with 9.7 μg of $TC^{O}TP$ (for in vitro incorporation experiments) or 100 ng of $tC^{O}$-labelled mRNA per 105 cells (for in vitro translation, cytotoxicity assessment, flow cytometry analysis and confocal microscopy), using a Neon Transfection System (Invitrogen, Carlsbad, CA, US) and following the protocol for 10 μL Neon Tip provided by the manufacturer, with a triple pulse of 1200 V and a pulse width of 20 ms. For chemical transfection, SH-SY5Y cells were seeded one day prior transfection at a density of 0.8 106 cells/mL, in 48-well plate or glass-bottomed culture dishes for flow cytometry or confocal microscopy analysis, respectively. Lipofectamine MessengerMAX was used as chemical reagent for transfection according to the manufacturer's instructions. Briefly, the reagent was diluted and incubated for 10 min at room temperature in Opti-MEM medium. The $tC^{O}$-mRNA constructs were added to the reagent to reach a 1:1 final ratio reagent-mRNA (v/w), followed by a 5 min incubation at room temperature allowing the complex mRNA-lipid to form. Cells were incubated with this complex up to 72 h. To address the impact of the dye incorporation on RNA translation, SH-SY5Y cells were electroporated or chemical transfected with commercially available non-labelled (NL) or Cyanine5-labelled (Cy5) eGFP encoding mRNAs (Trilink®) has described here.

**Cytotoxicity assessment**

Cell membrane integrity was determined using the Pierce™ LDH Cytotoxicity Assay Kit (Invitrogen) according to the manufacturer's instructions. Briefly, LDH released in the supernatants of cells 24 h post-electroporated or post-transfected with $tC^{O}$-labelled mRNA, or Cy5-mRNA, was measured with a coupled enzymatic assay which results in the conversion of a tetrazolium salt into a red formazan product. The absorbance was recorded at 490 nm and 680 nm. The toxicity was expressed as the percentage of LDH release in supernatant compared to maximum LDH release (supernatant + cell lysate). Data are means ± SD from three experiments performed in triplicate.

**Flow cytometry**

Following electroporation of $tC^{O}$-labelled mRNA, cells were seeded in 48-well plate (2.105 cells/well) and the expression of H2B:GFP in cells was quantified by flow cytometry. Briefly, 24 h, 48 h or 72 h post-electroporation or post-transfection with $tC^{O}$-mRNA, non-labelled mRNA or Cy5-mRNA, cells were harvested and analysed on a Guava EasyCyte 8HT flow cytometer (Millipore). Data are mean fluorescence intensities ± SD of gated single living cells from three experiments performed in triplicate. The average fluorescence intensities were baseline corrected by subtracting the signal for RNase-free water electroporated or transfected cells. All flow cytometry data were analysed in Flowing software (version 2.5.1) and displayed using R (http://www.R-project.org/). H2B:GFP: Excitation 488 nm; Emission 525-530 nm.

**Confocal microscopy**

After electroporation, cells were seeded in glass-bottomed culture dishes (MatTek glass-bottomed or in 4-sectors subdivided CELLview dishes; 2.105 cells/chamber). For tracking experiment, the Huh-7 cells stably overexpressing mRFP-Rab5 were incubated with lipofectamine/$tC^{O}$-mRNA complex and time-lapse was recorded up to 20 h post-chemical transfection. Confocal images were acquired on a Nikon C2+ confocal microscope equipped with a C2-DUVB GaAsP Detector Unit and using an oil-immersion 60 × 1.4 Nikon APO objective (Nikon Instruments, Amsterdam, Netherlands). Data were processed with the Fiji software. H2B:GFP: Excitation 488 nm; Emission 495-558 nm. tCO-labelled mRNA: Exc. 405 nm; Em. 447-486 nm. Cy5-labelled mRNA: Exc. 640 nm; Em. 652-700 nm. mRFP-Rab5: Exc. 561 nm; Em. 565-720 nm.

**Cell-free translation**

Cell-free translation reactions were performed using E. coli bacterial lysates and an Expressway™ Mini Cell-Free Expression System (Thermo Scientific). Calmodulin-like 3 protein is provided as a positive control plasmid (pEXP5-NT/CALML3) in the kit itself; this DNA vector contains a T7 polymerase promoter and a 6xHis tag, hence it was first in-vitro-transcribed in presence of the desired concentrations of $TC^{O}TP$ (vide supra). The obtained RNAs, once purified, were used as templates for the cell-free translation reaction according to the manufacturer's recommendations: E. coli slyD- Extract - 20 μl; 2.5X IVPS E. coli Reaction Buffer (-A.A.) - 20 μl; 50 mM Amino Acids (-Met) - 1.25 μ!; 75 mM Methionine* -1 μ!; T7 Enzyme Mix -1 μl (omitted when using $tC^{O}$-labelled RNAs); DNA Template - 1 μg (when testing the $tC^{O}$-labelled RNAs, added the same amount of RNA instead); DNase/RNase-free distilled water qsp 50 μl.

**Coomassie staining and Western Blots**

Protein samples from in vitro translation experiments were quantified with a Qubit Protein Assay kit (Thermo Scientific), mixed with 6x SDS Laemmli reducing buffer (Alfa Aesar), then heat-denatured at 85 °C for 10 min and kept at room temperature until needed. Samples were generally run in 1 mm polyacrylamide 4-20 % Novex MES/SDS gels (Thermo Scientific) and using a Mini Gel Tank, with the PSU set at constant voltage (200 V). For Coomassie staining, the gel was then washed three times in boiling water, to remove excess of SDS, on a benchtop shaker; a 1x Coomassie non-toxic staining solution was added to the gel and microwaved until initial boiling.

Gel was finally washed after the appropriate incubation time, to remove excess of background noise, in distilled water and imaged using a ChemiDoc Touch. For Western Blot, the gels were blotted onto PVDF LF ethanol-activated membranes (BioRad) with a TransBlot semi-dry system (BioRad), according to manufacturer's recommendations (settings for 1mm-thick gels and mixed weight proteins). PVDF membranes were then washed 5 min in TBS-T (TBS and 0,1 % Tween-20, Sigma Aldrich), blocked in 5 % milk in TBS for 1 h at room temperature and incubated with the appropriate primary antibody dilutions.

After 3x5 min washes in TBST and an incubation of 1 h with the corresponding HRP-conjugated secondary antibodies, the membrane was washed again three times in TBS-T, once in TBS and once more in distilled water. Finally, membranes were incubated with a minimal volume of SuperSignal West Pico PLUS (Thermo Scientific) and imaged with a ChemiDoc Touch. Primary antibodies: mouse monoclonal anti-6xHistidine tag (Invitrogen) and mouse monoclonal anti-GAPDH (ref. 437000, Invitrogen), both diluted 1:1000 in 3 % BSA/TBS-T. Secondary antibodies: HRP-conjugated polyclonal goat anti-Ms and anti-Rb Cross-Adsorbed IgG (H+L) (ref. A16072 and A16104, Invitrogen), used at 1:10000 dilution in TBS-T.

*Example 8: Spectroscopic Methods*

The $tC^{O}$-RNA products from the cell-free transcription reactions (prior to polyadenylation and capping, see Methods: Bio for details) were measured as received, i.e. in RNAse free Milli Q water. All measurements were carried out at room temperature (*ca.* 22°C) in a 3.0 mm path length quartz cuvette, with a sample volume of *ca*. 60 μL.

**Steady state absorption**

Absorption spectra were recorded on a Cary 5000 (Varian Technologies) spectrophotometer with a wavelength interval of 1.0 nm, integration time of 0.1 s, and a spectral band width (SBW) of 1 nm. All spectra were baseline corrected by subtracting the corresponding absorption from the solvent only. A second-order polynomial Savitzky-Golay (five points) smoothing filter was applied to all spectra. For samples exhibiting significant scattering, as evidenced by characteristic absorption in the long wavelength region (here for $\lambda > 475$ nm), an additional correction was applied. The scattering contribution ($A_{scatter}$) to the absorption was in such cases fitted (using absorption at 550-475 nm as input) to the Rayleigh scattering function (equation S1), where c is a proportionality constant and $A_0$ a constant, and then subtracted for all wavelengths.

$$A_{scatter}(\lambda) = log\left(\frac{1}{1 - c\times\lambda^{-4}}\right) + A_0 \qquad (S1)$$

**Steady state emission**

Emission spectra were recorded on a SPEX Fluorolog (Jobin Yvon Horiba) fluorimeter with excitation at 356 nm. Emission was collected at a right angle with an integration time of 0.1 s and wavelength interval of 1 nm. Monochromator slits were adjusted to achieve optimal signal output, leading to SBWs in the interval 1.5-2.5 nm on both the excitation and emission side. Emission spectra were corrected for Raman scattering by subtracting the corresponding emission from a sample containing only solvent. A second-order polynomial Savitzky-Golay (five points) smoothing filter was applied to all spectra.

**Fluorescence quantum yield determination**

Sample fluorescence quantum yields ($\Phi_F$) were determined relative to a solution of quinine sulphate (Sigma) in 0.5 M $H_2SO_4$ ($\Phi_{F,REF}$ = 0.546) and calculated according to equation S2.

$$\Phi_F = \Phi_{F,REF} \times \frac{\int_{\lambda_i}^{\lambda_f} I_S(\lambda)d\lambda}{\int_{\lambda_i}^{\lambda_f} I_{REF}(\lambda)d\lambda} \times \frac{A_{REF}}{A_S} \times \frac{\eta_S^2}{\eta_{REF}^2} \qquad \text{(S2)}$$

Emission spectra for the sample, $I_S(\lambda)$and reference, $I_{REF}(\lambda)$, were integrated between $\lambda_i$ = 365 nm and $\lambda_f$ = 700 nm. Absorption at the excitation wavelength (356 nm) for the sample ($A_s$) and reference ($A_{REF}$) were in the interval 0.05-0.11 for all samples. Adopted solvent refractive indices for the samples (water) and reference (0.5 M $H_2SO_4$) were $\eta_S$ = 1.333 and $\eta_{REF}$ = 1.339, respectively. All quantum yields are presented as mean ± standard deviation of two independent cell-free transcription reactions.

**Time-resolved emission**

Fluorescence lifetimes were determined using time-correlated single photon counting (TCSPC). Samples were excited using an LDH-P-C-375 (PicoQuant) pulsed laser diode with emission centred at 377 nm (FWHM pulse width was 1 nm and 70 ps with respect to wavelength and time, respectively), operated with a PDL 800-B (PicoQuant) laser driver at a repetition frequency of 10 MHz. Sample emission (458 nm, SBW = 10 nm) was collected at a right angle, through an emission polarizer set at 54.9° (magic angle detection). Photon counts were recorded on a R3809U 50 microchannel plate PMT (Hamamatsu) and fed into a LifeSpec multichannel analyser (Edinburgh Analytical Instruments) with 2048 active channels (24.4 ps/channel), until the stop condition of 104 counts in the top channel was met. The instrument response function (IRF) was determined using a frosted glass (scattering) modular insert while observing the emission at 377 nm (SBW = 10 nm).

**Fitting of fluorescence lifetimes**

The intensity decays were fitted with IRF re-convolution to the multiexponential model shown in equation S3.

$$I(t) = \int_0^t IRF(t') \sum_{i=1}^{n} \alpha_i e^{-\frac{t-t'}{\tau_i}} dt' \qquad \text{(S3)}$$

The least-square re-convolution fitting procedure was carried out using the DecayFit software (http://www.fluortools.com/software/decayfit). All decays were fitted to a tri-exponential (n = 3) model. The presented lifetimes are amplitude-weighted average lifetimes ($\bar{\tau}$), calculated using the pre-exponential factors $\alpha_i$ and lifetimes ($\tau_i$) according to equation S4. The fitting parameters for the decays are shown in Table 2.

$$\bar{\tau} = \sum_{i=1}^{n} \alpha_i \tau_i \qquad \text{(S4)}$$

**Table 2**: Fitted lifetime parameters for the TCSPC experiments. The $X^2$-value (Chi-Square) was evaluated to indicate goodness of fit.

| | transcript | $\alpha_1$ | $\tau_1$ (ns) | $\alpha_2$ | $\tau_2$ (ns) | $\alpha_3$ | $\tau_3$ (ns) | $\bar{\tau}$ (ns) | $X^2$ |
|---|---|---|---|---|---|---|---|---|---|
| Set 1 | 25% | 0.19 | 0.67 | 0.49 | 3.2 | 0.31 | 5.6 | 3.5 | 1.09 |
| | 50% | 0.28 | 0.71 | 0.43 | 2.8 | 0.30 | 5.2 | 2.9 | 1.06 |
| | 75% | 0.33 | 0.68 | 0.45 | 2.6 | 0.23 | 5.2 | 2.5 | 1.12 |
| | 100% | 0.33 | 0.48 | 0.44 | 2.1 | 0.23 | 4.8 | 2.2 | 1.00 |
| Set 2 | 25% | 0.21 | 0.63 | 0.40 | 2.8 | 0.34 | 5.3 | 3.3 | 0.99 |
| | 50% | 0.26 | 0.64 | 0.41 | 2.7 | 0.30 | 5.2 | 3.0 | 1.03 |
| | 75% | 0.29 | 0.50 | 0.42 | 2.1 | 0.22 | 4.8 | 2.4 | 1.02 |
| | 100% | 0.37 | 0.47 | 0.40 | 1.9 | 0.39 | 4.5 | 2.0 | 1.01 |

**Cell-free transcription reaction kinetics**

The ratio of the rate constants for cytosine vs. $tC^O$ incorporation ($k_C/k_{tC0}$) was calculated using the absorption spectra of the $tC^O$-RNA transcripts ($A_{260}$ and $A_{369}$), and triphosphate initial concentrations ($[CTP]_0$ and $[tC^OTP]_0$) as input. Equations S5 and S6 follows upon assuming first order reaction kinetics with respect to the triphosphate species [CTP] and [$tC^O$TP].

$$\frac{d[\mathrm{CTP}]}{dt} = -k_C \times [\mathrm{CTP}] \quad \text{(S5)}$$

$$\frac{d[tC^O\mathrm{TP}]}{dt} = -k_{tC^0} \times [tC^O\mathrm{TP}] \quad \text{(S6)}$$

Solving S5 and S6 for the respective rate constants renders equation S7, in which [C] and [tC°] denote the concentration of incorporated C and $tC^O$, respectively.

$$\frac{k_C}{k_{tC^0}} = \frac{ln\left(\frac{[CTP]_0}{[CTP]_0-[C]}\right)}{ln\left(\frac{[tC^OTP]_0}{[tC^OTP]_0-[tC^O]}\right)} \quad \text{(S7)}$$

Using the Lambert-Beer law, absorption is related to nucleobase concentration according to equations S8 and S9. The following molar absorptivities (unit: $M^{-1}cm^{-1}$) were adopted:

$\varepsilon_{260}^{tC^O}$ = 12200, $\varepsilon_{260}^{C}$ = 7400, $\varepsilon_{260}^{G}$ = 11800, $\varepsilon_{260}^{U}$ = 9300, $\varepsilon_{260}^{A}$ = 15300, $\varepsilon_{369}^{tC^O}$ = 9370.

$$A_{260} = 0.9 \times l \times (\varepsilon_{260}^{tC^O} \times [tC^0] + \varepsilon_{260}^{C} \times [C] + \varepsilon_{260}^{G} \times [G] + \varepsilon_{260}^{U} \times [U] + \varepsilon_{260}^{A} \times [A]) \quad \text{(S8)}$$

$$A_{369} = l \times \varepsilon_{369}^{tC^O} \times [tC^0] \quad \text{(S9)}$$

Assuming that the product RNA is uniform in size (1247 nucleotides), its base composition (A: 408, U: 272, G: 307, C: 260) allows for equations S10 - S13.

$$[tC^0] + [C] = [RNA] \times 260 \quad \text{(S10)}$$

$$[A] = [RNA] \times 408 \quad \text{(S11)}$$

$$[U] = [RNA] \times 272 \quad \text{(S12)}$$

$$[G] = [RNA] \times 307 \quad \text{(S13)}$$

Solving the equation system composed of S7 through S13 allows for quantification of $k_C/k_{tC0}$, $[tC^0]$, $[RNA]$, $[C]$, $[A]$, $[U]$, and $[G]$. The average-strand $tC^0$ incorporation degree ($\theta_{tCO}$) can then be calculated according to equation S14.

$$\theta_{tC^O} = \frac{[tC^0]}{[C]+[tC^0]} \quad \text{(S14)}$$

Using the volume of the cell-free reaction (50 µL) and resulting product solution (100 µL), equation S15 was applied to calculate the $tC^0$ incorporation yield ($\eta_{tC0}$).

$$\eta_{tC^0} = \frac{[tC^0] \times 100\ \mu L}{[tC^0TP]_0 \times 50\ \mu L} \quad \text{(S15)}$$

Consequently, the RNA yield ($\eta_{RNA}$) was calculated according to equation S16.

$$\eta_{RNA} = \frac{[RNA] \times 100\ \mu L}{[tC^0TP]_0 \times 50\ \mu L} \quad \text{(S16)}$$

*Conclusions*

This specification demonstrates that an artificial, size-expanded analogue of cytosine takes the role of natural cytosine and is correctly recognized by several enzymatic machineries, including the ribosome. This fluorescent base analogue, $tC^O$, is demonstrated to be a suitable intrinsic imaging label of different size RNAs which minimally perturbs native properties and is compatible with enzymatic labelling processes.

Modified transcripts are non-toxic and translationally active both in bacterial lysate and in eukaryotic systems, regardless of their degree of $tC^O$ incorporation. This conveniently allows for simultaneous monitoring of mRNA uptake and translation into H2B:GFP in live-cell confocal microscopy using selective excitation, an approach that should be applicable to the translation of any protein similarly tagged with a GFP family protein.

The intrinsic fluorescence RNA-labelling methodologies disclosed herein are therefore excellent non-invasive ways to, in real time, elucidate cellular trafficking mechanisms such as endosomal escape or exosomes formation, both of which are of fundamental importance for pharmaceutical applications. As such the technology for live cell imaging should enable new and improved delivery strategies for next-generation nucleic acid-based drugs.

## Claims

**1.** A compound of formula (I), also known as $tC^OTP$, or a salt thereof:

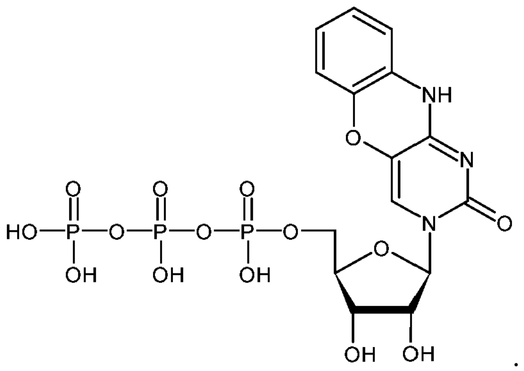

(I)

**2.** A compound of formula (I) as claimed in claim 1.

**3.** A compound of formula (I) as claimed in claim 1 which is a sodium, potassium, or ammonium salt.

**4.** A compound of formula (I) as claimed in claim 3 which is a monosodium, disodium, trisodium, monoammonium, diammonium or triammonium salt.

**5.** A process for preparing a compound of formula (I) or a salt thereof as claimed in any of the preceding claims comprising:

i. Providing a compound of formula (II) or a salt thereof:

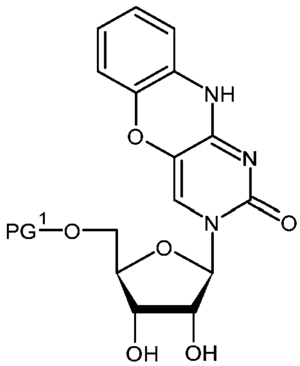

(II)

Where $PG^1$ is a suitable protecting group;
ii. Immobilising the compound of formula (II) or a salt thereof by linking one of its secondary alcohol groups to a suitable support;
iii. Capping any free secondary alcohol groups with a suitable protecting group $PG^2$;
iv. Removing the protecting group $PG^1$;
v. Reacting the exposed primary alcohol group with a compound of formula (III):

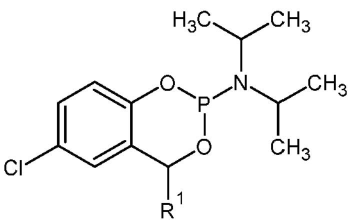

(III)

Where $R^1$ is selected from a hydro group and a $C_{1-3}$alkyl group;
vi. Oxidising the resultant phosphorus (III) compound to a phosphorus (V) compound;
vii. Reacting the phosphorus (V) compound with a tetraalkylammonium pyrophosphate to generate a triphosphate;
viii. Removing the protecting group $PG^2$; and
ix. Cleaving the resultant triphosphate from the support to generate a compound of formula (I) or salt thereof.

**6.** A process for preparing a compound of formula (I) or a salt thereof as claimed in claim 5, where $R^1$ is a methyl group.

**7.** A process for preparing a compound of formula (I) or a salt thereof as claimed in claim 5 or claim 6, where the support is a solid polymer.

**8.** A process for preparing a compound of formula (I) or a salt thereof as claimed in any of claims 5 to 7, where $PG^1$ is dimethoxytrityl and $PG^2$ is acetyl.

**9.** A composition for preparing a tC$^O$ labelled RNA molecule comprising a compound of formula (I) as claimed in any of claims 1 to 4 and a natural ribonucleotide triphosphate.

**10.** The use of a compound of formula (I) or a salt thereof as claimed in any of claims 1 to 4 to enzymatically prepare a tC$^O$ labelled RNA molecule.

**11.** The use of a compound of formula (I) or a salt thereof as claimed in claim 10 where the RNA molecule is mRNA.

**12.** A process for preparing a tC$^O$ labelled RNA molecule comprising providing a DNA template to a composition comprising a compound of formula (I) and a natural ribonucleotide triphosphate, then treating the resultant mixture with an RNA polymerase.

**13.** The use of a tC$^O$ labelled mRNA molecule to prepare a protein encoded by the mRNA by translation.

**14.** The use of a tC$^O$ labelled mRNA molecule as claimed in claim 13, where the encoded protein is fused to a fluorescent protein.

**15.** The use of a tC$^O$ labelled mRNA molecule as claimed in claim 14, where the tC$^O$ labelled mRNA and the encoded protein are simultaneously analysed spatiotemporally using confocal microscopy.

**Patentansprüche**

**1.** Verbindung der Formel (I), auch als TC$^0$TP bekannt, oder ein Salz davon:

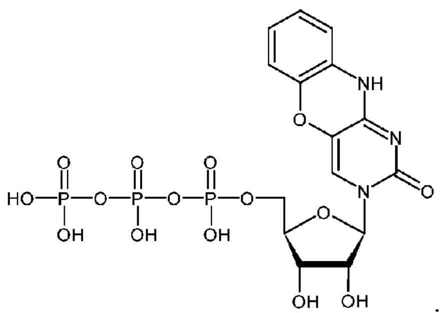

(I)

**2.** Verbindung der Formel (I) nach Anspruch 1.

**3.** Verbindung der Formel (I) nach Anspruch 1, die ein Natrium-, Kalium- oder Ammoniumsalz ist.

**4.** Verbindung der Formel (I) nach Anspruch 3, die ein Mononatrium-, Dinatrium-, Trinatrium-, Monoammonium-, Diammonium- oder Triammoniumsalz ist.

5. Verfahren zur Herstellung einer Verbindung der Formel (I) oder eines Salzes davon nach einem der vorstehenden Ansprüche, das Folgendes umfasst:

i. Bereitstellen einer Verbindung der Formel (II) oder eines Salzes davon:

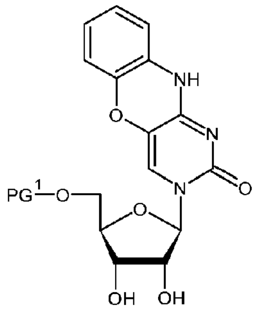

(II) ,

wo $PG^1$ eine geeignete Schutzgruppe ist;
ii. Immobilisieren der Verbindung der Formel (II) oder eines Salzes davon durch Verknüpfen einer deren sekundären Alkoholgruppen mit einem geeigneten Träger;
iii. Capping jedweder freien sekundären Alkoholgruppen mit einer geeigneten Schutzgruppe $PG^2$;
iv. Entfernen der Schutzgruppe $PG^1$;
v. Reagieren der ungeschützten primären Alkoholgruppe mit einer Verbindung der Formel (III):

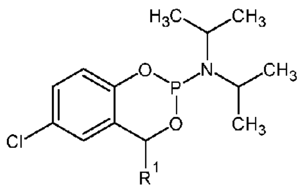

(III) ,

wo $R^1$ aus einem Wasserstoffatom und einer $C_{1-3}$-Alkylgruppe ausgewählt ist;
vi. Oxidieren der resultierenden Phosphor(III)-Verbindung zu einer Phosphor(V)-Verbindung;
vii. Reagieren der Phosphor (V)-Verbindung mit einem Tetraalkylammoniumpyrophosphat, um ein Triphosphat zu erzeugen;
viii. Entfernen der Schutzgruppe $PG^2$; und
ix. Abspalten des resultierenden Triphosphats von dem Träger, um eine Verbindung der Formel (I) oder ein Salz davon zu erzeugen.

6. Verfahren zur Herstellung einer Verbindung der Formel (I) oder eines Salzes davon nach Anspruch 5, wo $R^1$ eine Methylgruppe ist.

7. Verfahren zur Herstellung einer Verbindung der Formel (I) oder eines Salzes davon nach Anspruch 5 oder Anspruch 6, wo der Träger ein festes Polymer ist.

8. Verfahren zur Herstellung einer Verbindung der Formel (I) oder eines Salzes davon nach einem der Ansprüche 5 bis 7, wo $PG^1$ Dimethoxytrityl ist und $PG^2$ Acetyl ist.

9. Zusammensetzung für die Herstellung eines $tC^0$-markierten RNA-Moleküls, die eine Verbindung der Formel (I) nach einem der Ansprüche 1 bis 4 und ein natürliches Ribonukleotidtriphosphat umfasst.

10. Verwendung einer Verbindung der Formel (I) oder eines Salzes davon nach einem der Ansprüche 1 bis 4 zur enzymatischen Herstellung eines $tC^0$-markierten RNA-Moleküls.

11. Verwendung einer Verbindung der Formel (I) oder eines Salzes davon nach Anspruch 10, wo das RNA-Molekül mRNA ist.

12. Verfahren zur Herstellung eines $tC^0$-markierten RNA-Moleküls, das Folgendes umfasst: Bereitstellen eines DNA-Templates für eine Zusammensetzung, die eine Verbindung der Formel (I) und ein natürliches Ribonukleotidtriphosphat umfasst, dann Behandeln der resultierenden Mischung mit einer RNA-Polymerase.

13. Verwendung eines $tC^0$-markierten RNA-Moleküls zur Herstellung eines Proteins, das von der mRNA kodiert wird, durch Translation.

14. Verwendung eines $tC^0$-markierten RNA-Moleküls nach Anspruch 13, wo das kodierte Protein an ein fluoreszierendes Protein fusioniert wird.

15. Verwendung eines $tC^0$-markierten RNA-Moleküls nach Anspruch 14, wo die $tC^0$-markierte mRNA und das kodierte Protein gleichzeitig unter Verwendung von konfokaler Mikroskopie raumzeitlich analysiert werden.

## Revendications

1. Composé de la formule (I), également connu sous le nom de tC°TP, ou un sel de celui-ci :

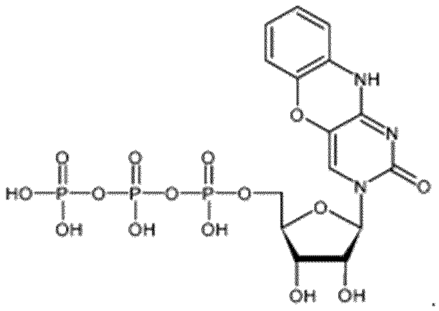

(I)

2. Composé de formule (I) selon la revendication 1.

3. Composé de la formule (I) selon la revendication 1, qui est un sel de sodium, de potassium ou d'ammonium.

4. Composé de formule (I) selon la revendication 3, qui est un sel de monosodium, disodium, trisodium, monoammonium, diammonium ou triammonium.

5. Procédé de préparation d'un composé de formule (I) ou d'un sel de celui-ci selon l'une quelconque des revendications précédentes, comprenant :

i. la mise à disposition d'un composé de formule (II) ou d'un sel de celui-ci :

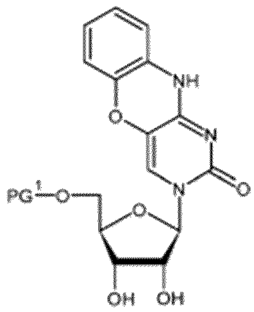

(II)

dans laquelle $PG^1$ est un groupement protecteur convenable ;
ii. l'immobilisation du composé de formule (II) ou d'un sel de celui-ci par la liaison de l'un de ses groupements d'alcool secondaire à un support convenable ;
iii. le coiffage de tout groupement d'alcool secondaire libre par un groupement protecteur convenable $PG^2$ ;
iv. l'élimination du groupement protecteur $PG^1$ ;
v. la réaction du groupement d'alcool primaire exposé avec un composé de formule (III) :

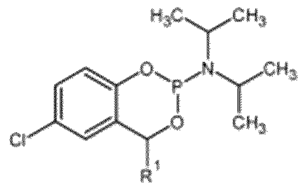

(III)

dans laquelle $R^1$ est choisi parmi un groupement hydro et un groupement $C_{1-3}$alkyle ;
vi. l'oxydation du composé de phosphore (III) résultant en un composé de phosphore (V) ;
vii. la réaction du composé de phosphore (V) avec un pyrophosphate de tétraalkylammonium, afin de générer un triphosphate ;
viii. l'élimination du groupement protecteur $PG^2$ ; et
ix. le clivage du triphosphate résultant du support, afin de générer un composé de formule (I) ou un sel de celui-ci.

**6.** Procédé de préparation d'un composé de formule (I) ou d'un sel de celui-ci selon la revendication 5, dans lequel $R^1$ est un groupement méthyle.

**7.** Procédé de préparation d'un composé de formule (I) ou d'un sel de celui-ci selon la revendication 5 ou la revendication 6, dans lequel le support est un polymère solide.

**8.** Procédé de préparation d'un composé de formule (I) ou d'un sel de celui-ci selon l'une quelconque des revendications 5 à 7, dans lequel $PG^1$ est diméthoxytrityle et $PG^2$ est acétyle.

**9.** Composition destinée à la préparation d'une molécule d'ARN marquée par tC°, comprenant un composé de formule (I) selon l'une quelconque des revendications 1 à 4 et un ribonucléotide triphosphate naturel.

**10.** Utilisation d'un composé de formule (I) ou d'un sel de celui-ci selon l'une quelconque des revendications 1 à 4, afin de préparer, par voie enzymatique, une molécule d'ARN marquée par tC°.

**11.** Utilisation d'un composé de formule (I) ou d'un sel de celui-ci selon la revendication 10, dans laquelle la molécule d'ARN est un ARNm.

**12.** Procédé de préparation d'une molécule d'ARN marquée par tC°, comprenant la mise à disposition d'une matrice d'ADN à une composition comprenant un composé de formule (I) et un ribonucléotide triphosphate naturel, puis le traitement du mélange résultant par une ARN polymérase.

**13.** Utilisation d'une molécule d'ARNm marquée par tC°, afin de préparer, par traduction, une protéine codée par l'ARNm.

**14.** Utilisation d'une molécule d'ARNm marquée par tC° selon la revendication 13, dans laquelle la protéine codée est fusionnée à une protéine fluorescente.

**15.** Utilisation d'une molécule d'ARNm marquée par tC° selon la revendication 14, dans laquelle l'ARNm marqué par tC° et la protéine codée sont analysés simultanément de manière spatio-temporelle par microscopie confocale.

**Figure 1**

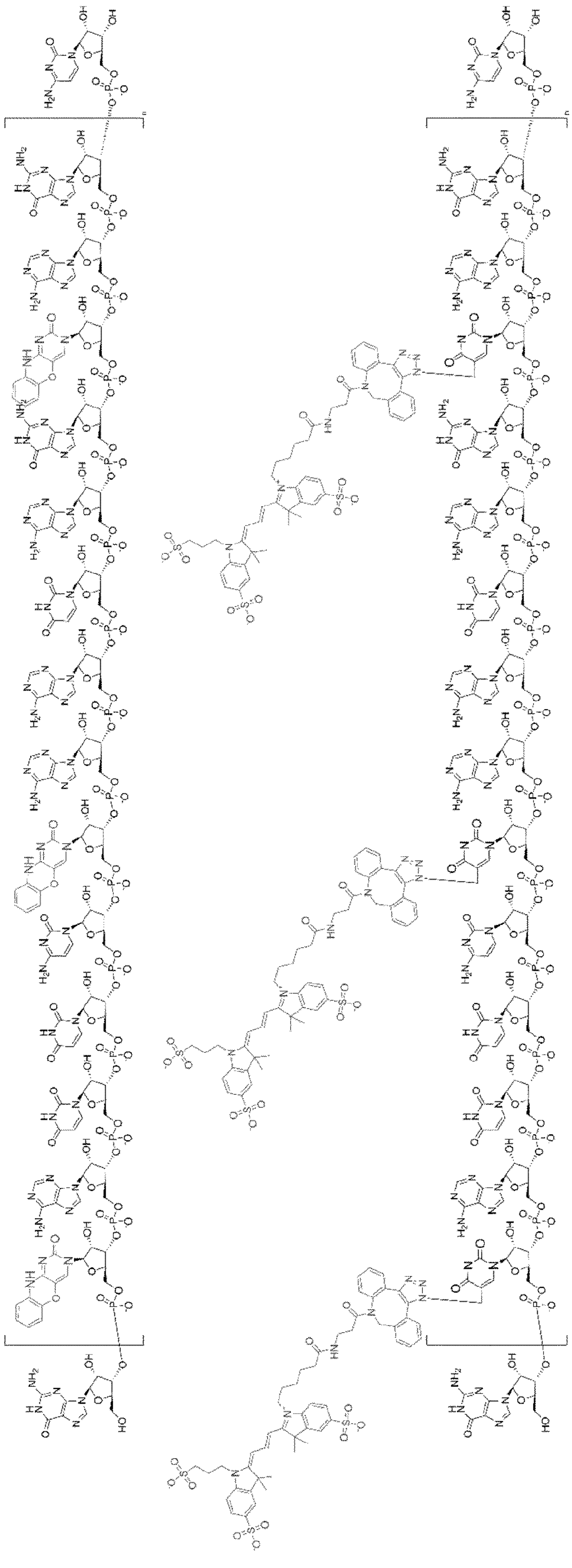

**Figure 2**

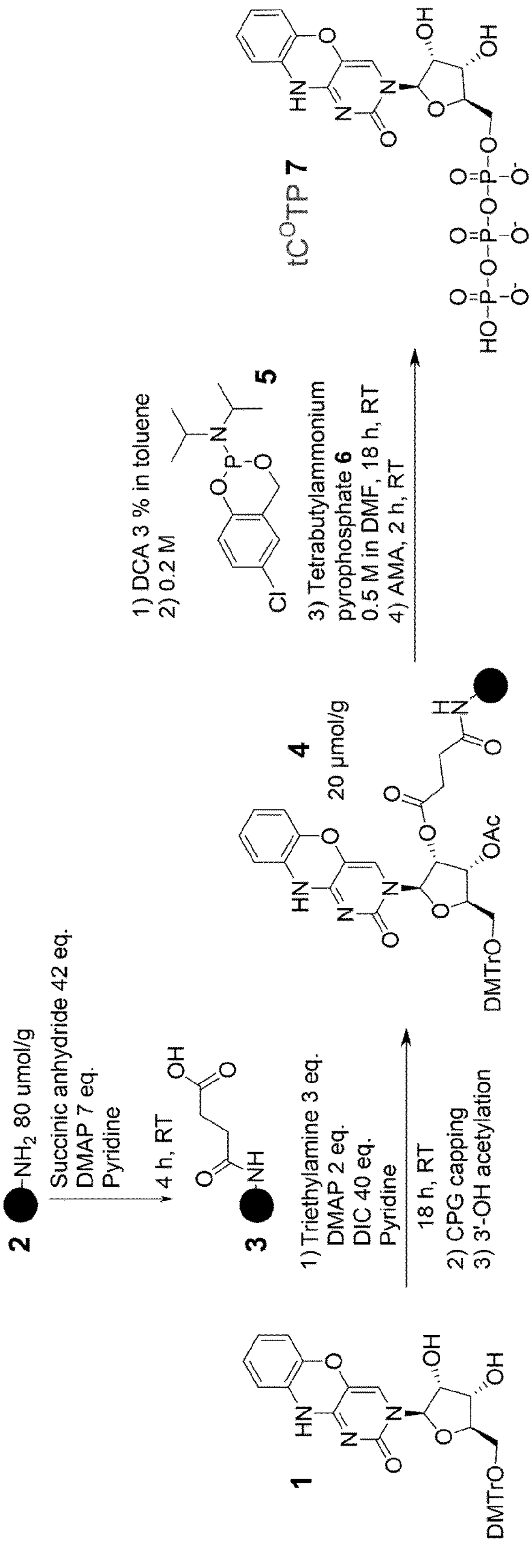
1
2
80 umol/g
Succinic anhydride 42 eq.
DMAP 7 eq.
Pyridine
4 h, RT
3
1) Triethylamine 3 eq.
DMAP 2 eq.
DIC 40 eq.
Pyridine
18 h, RT
2) CPG capping
3) 3'-OH acetylation
4
20 µmol/g
1) DCA 3 % in toluene
2) 0.2 M
5
3) Tetrabutylammonium
pyrophosphate 6
0.5 M in DMF, 18 h, RT
4) AMA, 2 h, RT
tC$^{O}$TP 7

Figure 3a

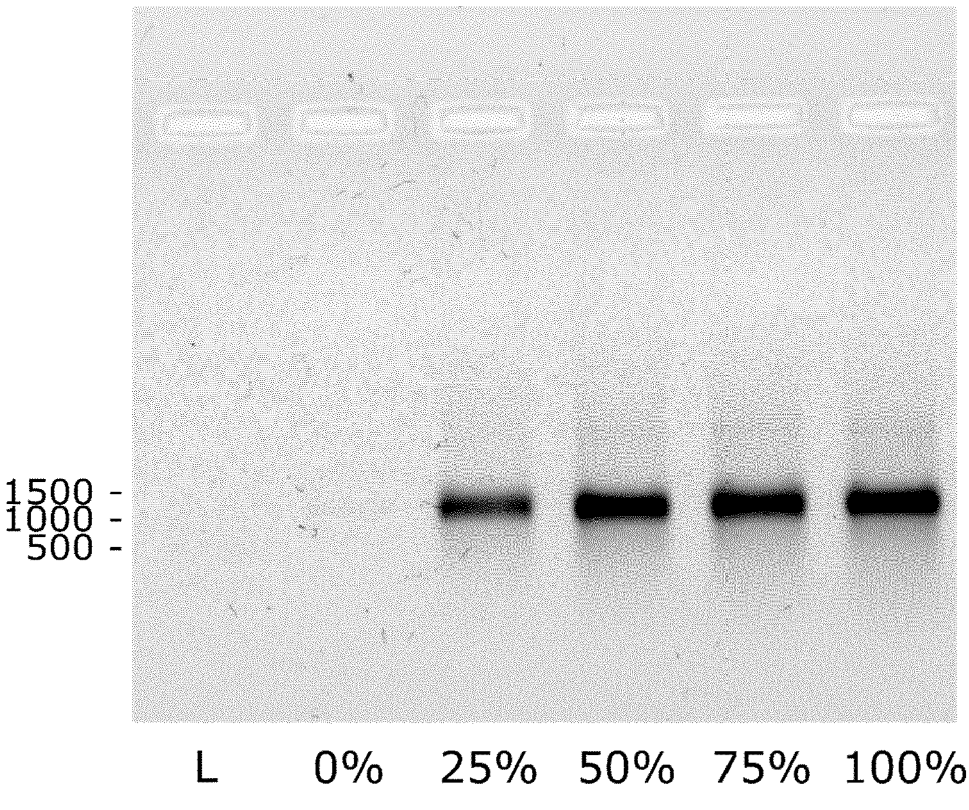

Figure 3b

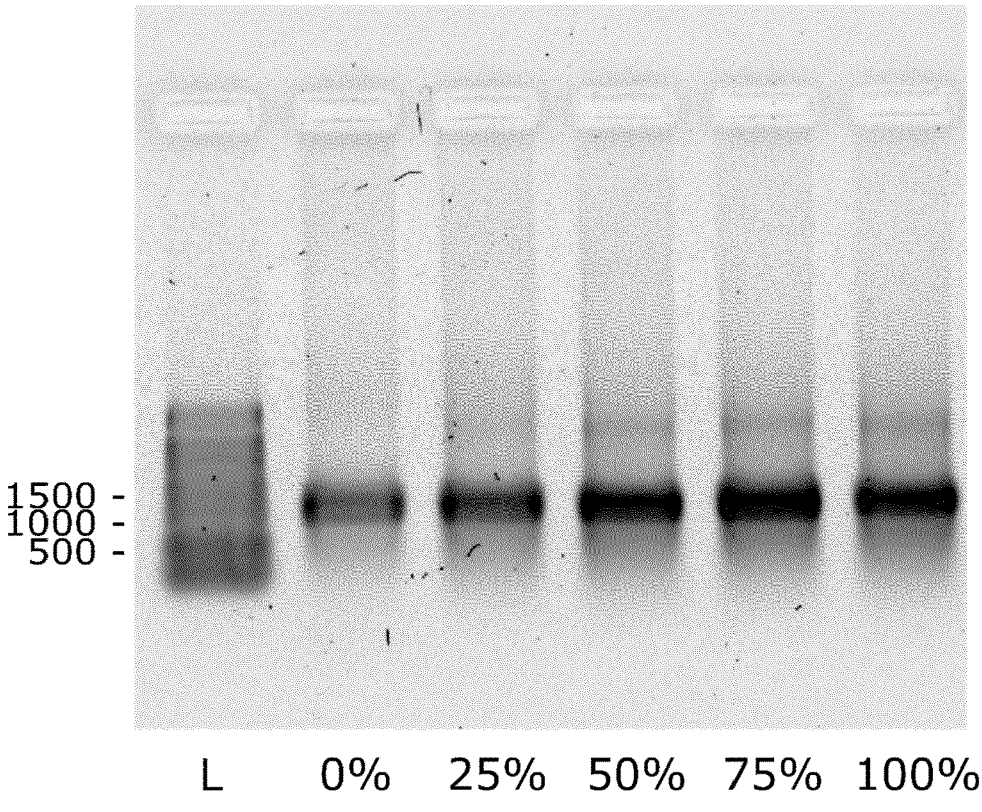

Figure 3c

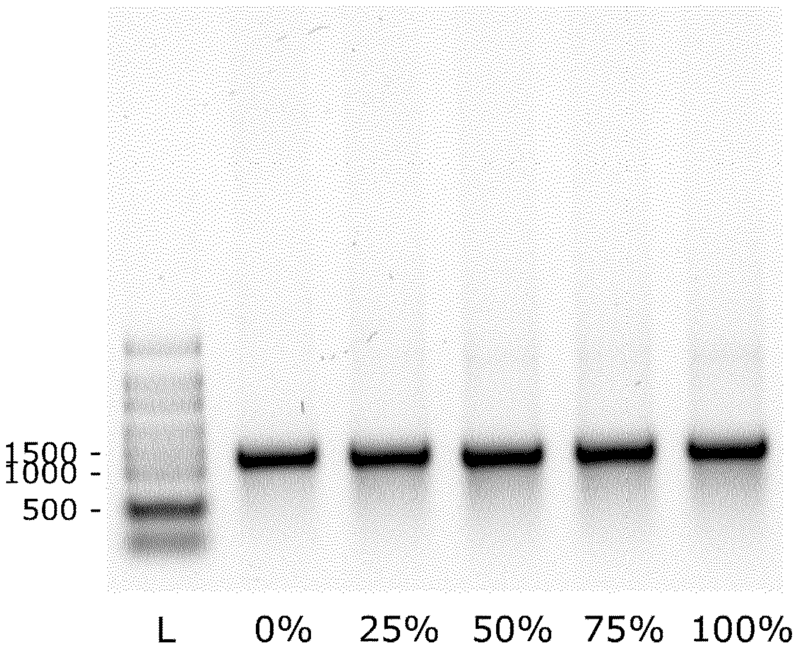

**Figure 4a**

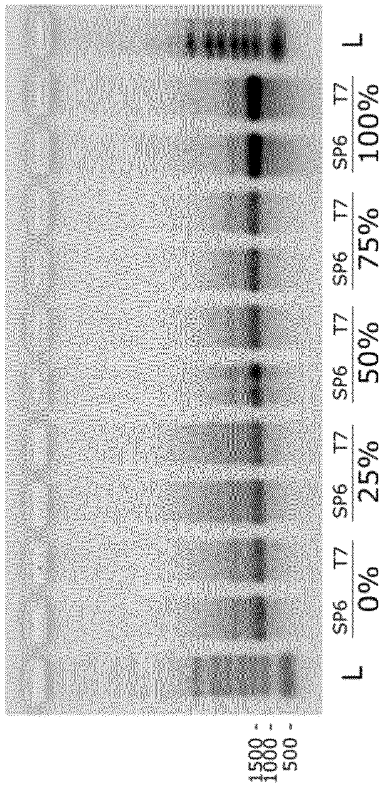

1500 -
1000 -
500 -
L
SP6
T7
0%
SP6
T7
25%
SP6
T7
50%
SP6
T7
75%
SP6
T7
100%
L


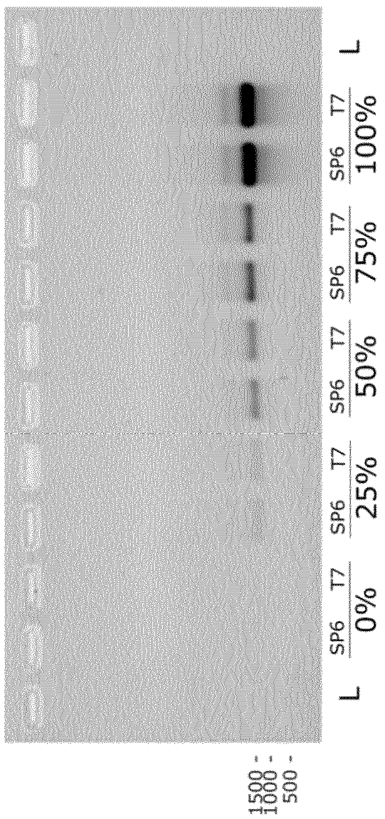

1500 -
1000 -
500 -
L
SP6
T7
0%
SP6
T7
25%
SP6
T7
50%
SP6
T7
75%
SP6
T7
100%
L

**Figure 4b**

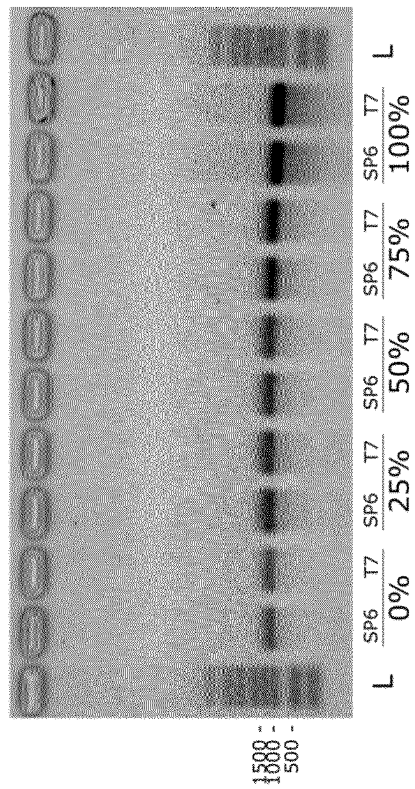
L
SP6
T7
0%
25%
50%
75%
100%
1500
1000
500

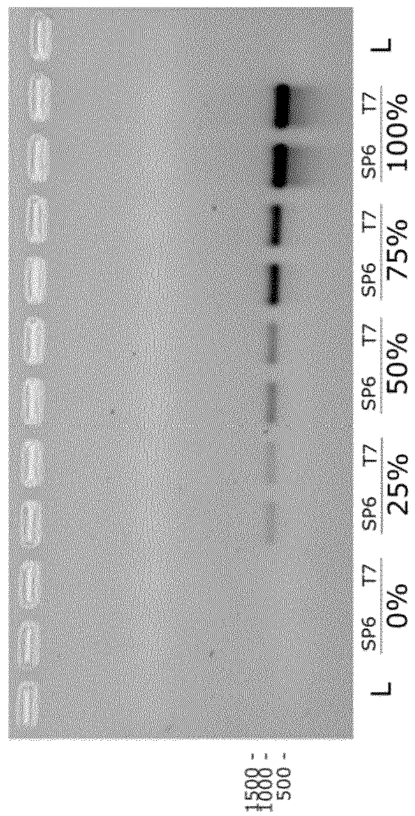
L
SP6
T7
0%
25%
50%
75%
100%
1500
1000
500

Figure 5a

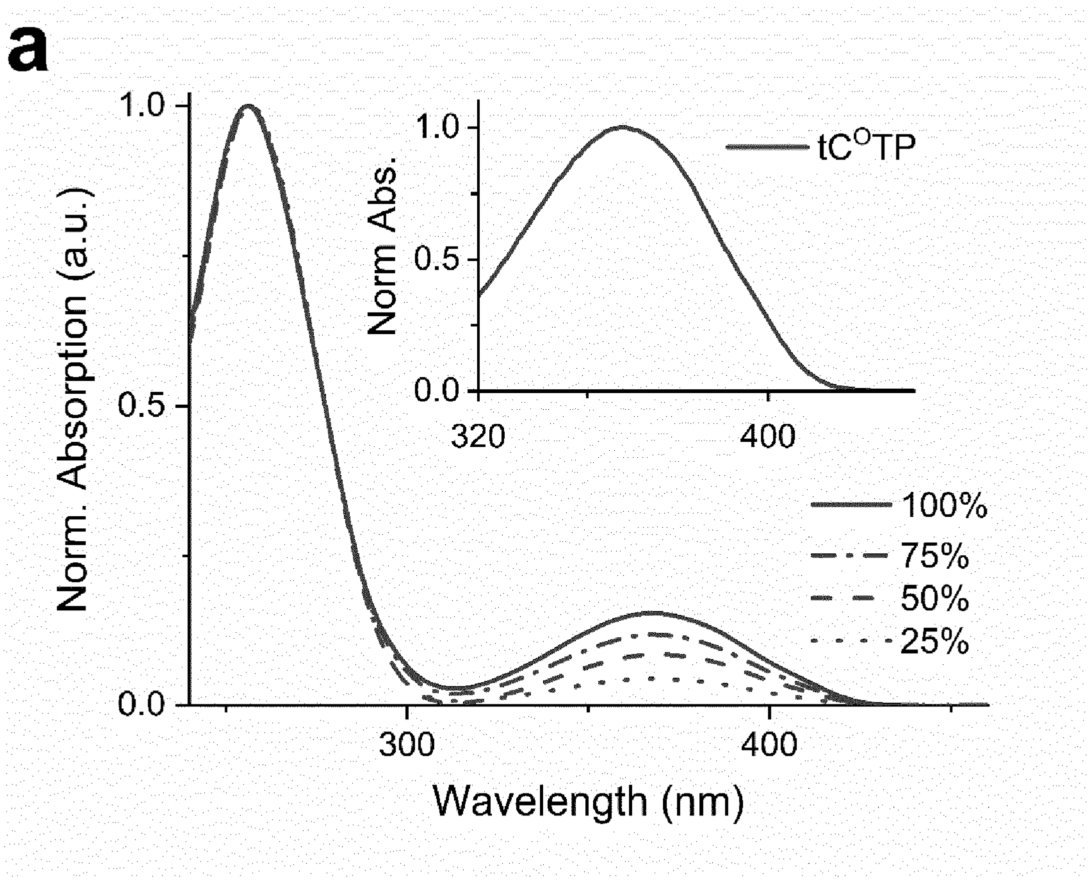
a
1.0
0.5
0.0
Norm. Absorption (a.u.)
300
400
Wavelength (nm)
1.0
0.5
0.0
Norm Abs.
320
400
tC$^{O}$TP
100%
75%
50%
25%

**Figure 5b**

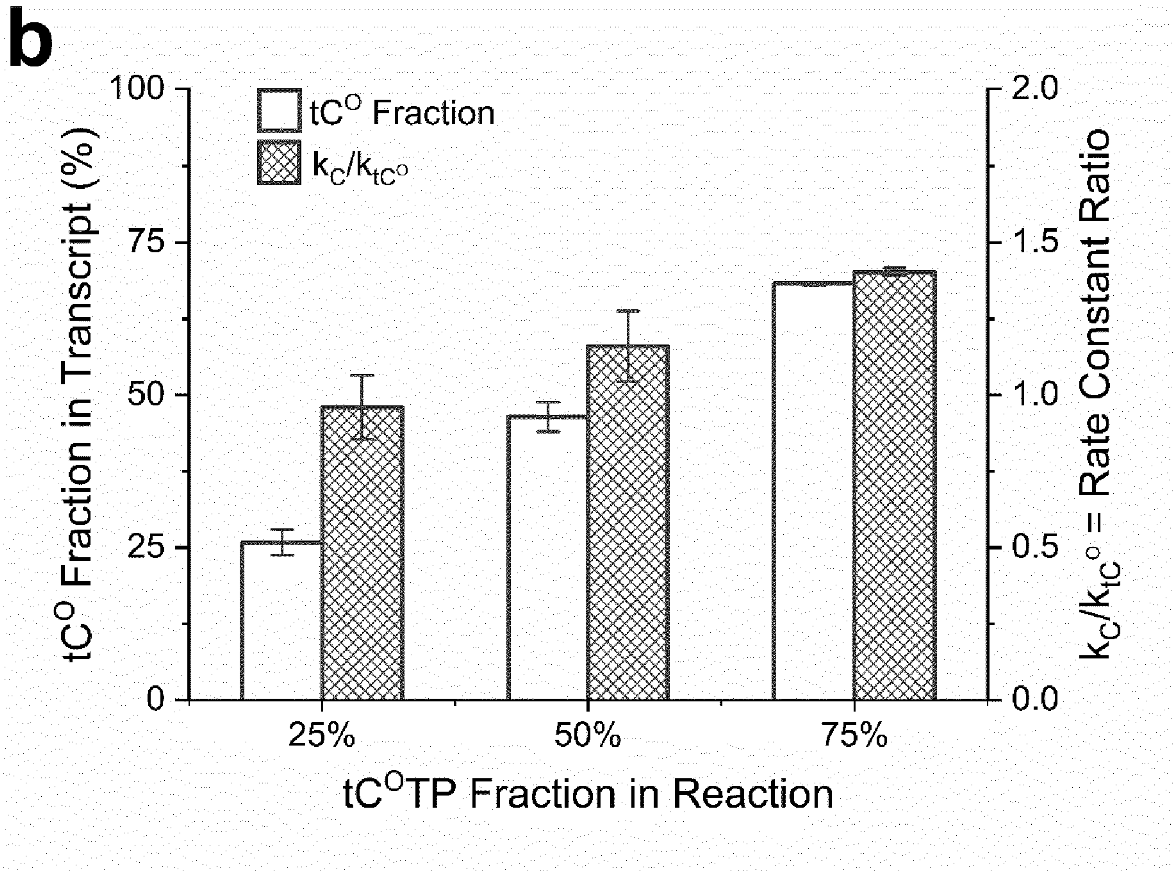
b
100
75
50
25
0
2.0
1.5
1.0
0.5
0.0
tC^O Fraction
k_C/k_tC^O
25%
50%
75%
tC^O Fraction in Transcript (%)
k_C/k_tC^O = Rate Constant Ratio
tC^OTP Fraction in Reaction

Figure 5c

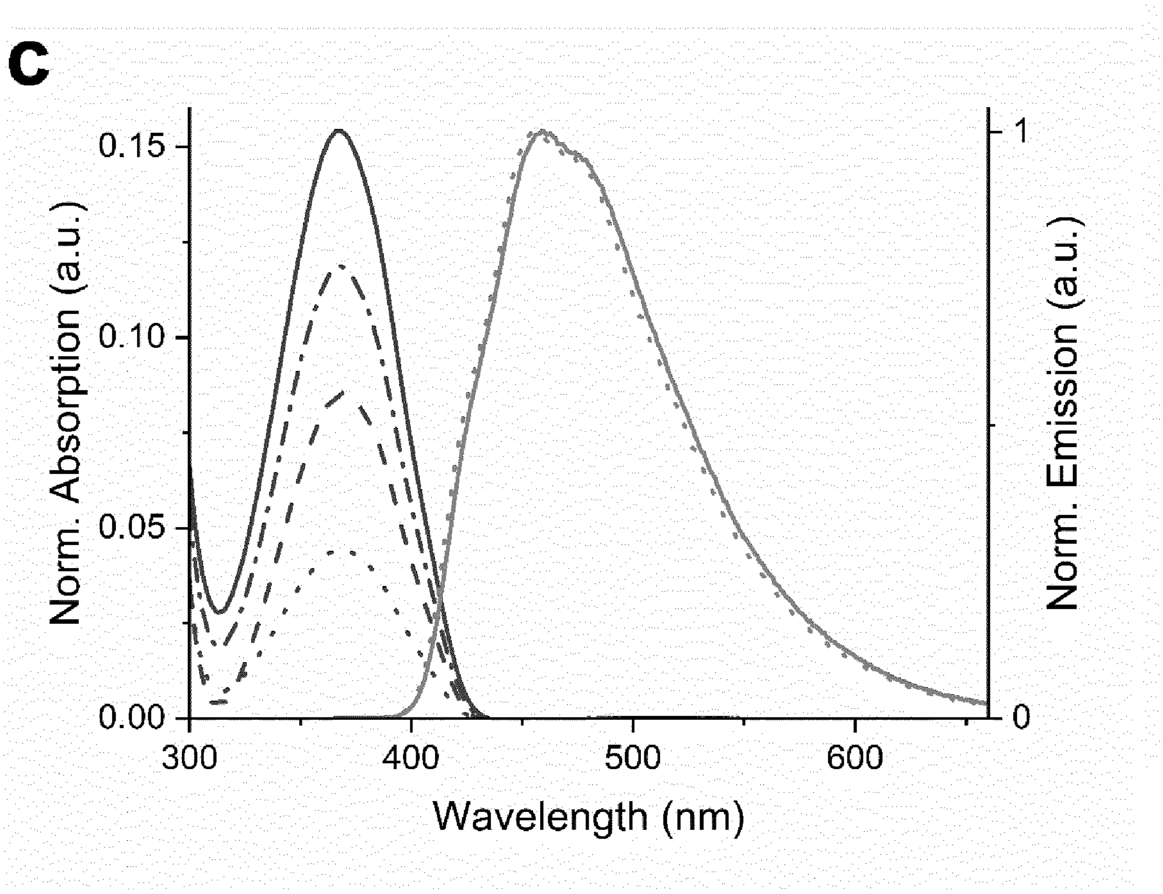
c
Norm. Absorption (a.u.)
0.15
0.10
0.05
0.00
300
400
500
600
Wavelength (nm)
Norm. Emission (a.u.)
1
0

Figure 5d

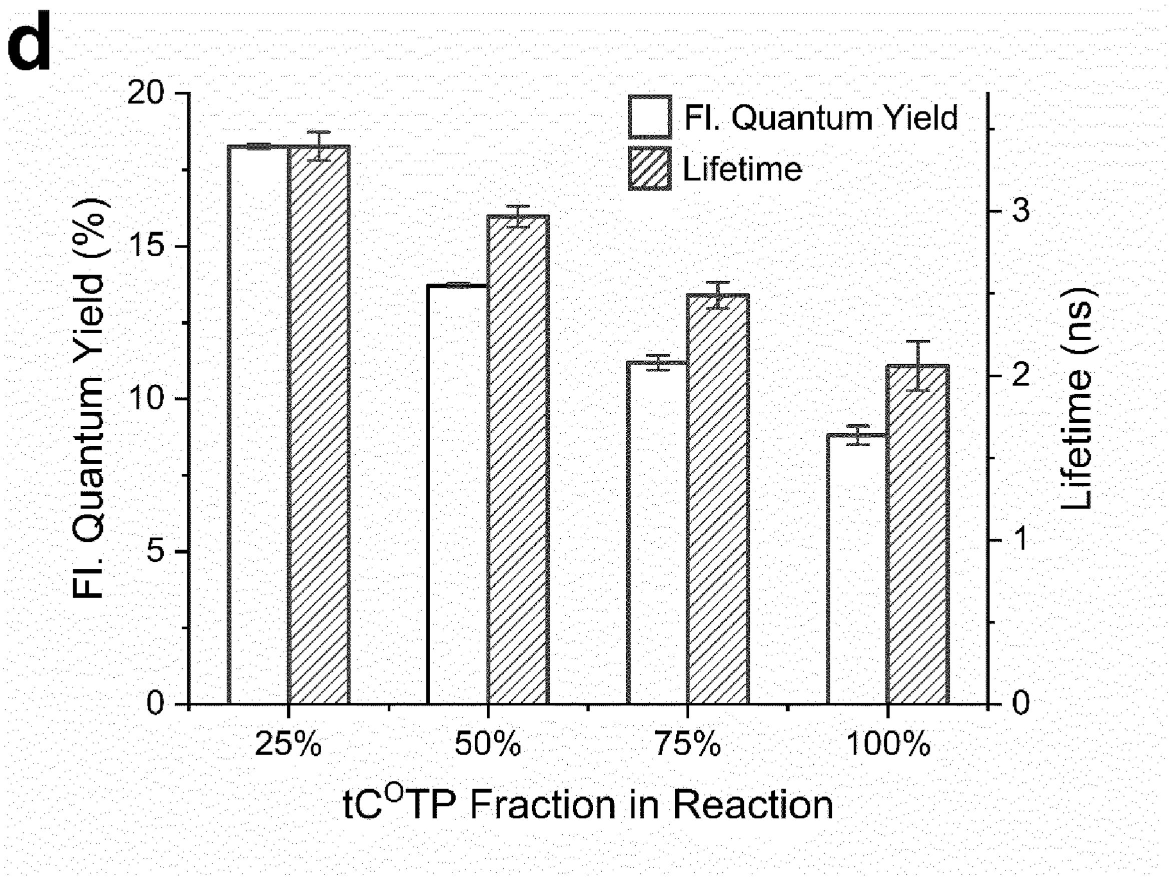
d
20
15
10
5
0
Fl. Quantum Yield (%)
Fl. Quantum Yield
Lifetime
3
2
1
0
Lifetime (ns)
25%
50%
75%
100%
tC$^{O}$TP Fraction in Reaction

**Figure 6a**

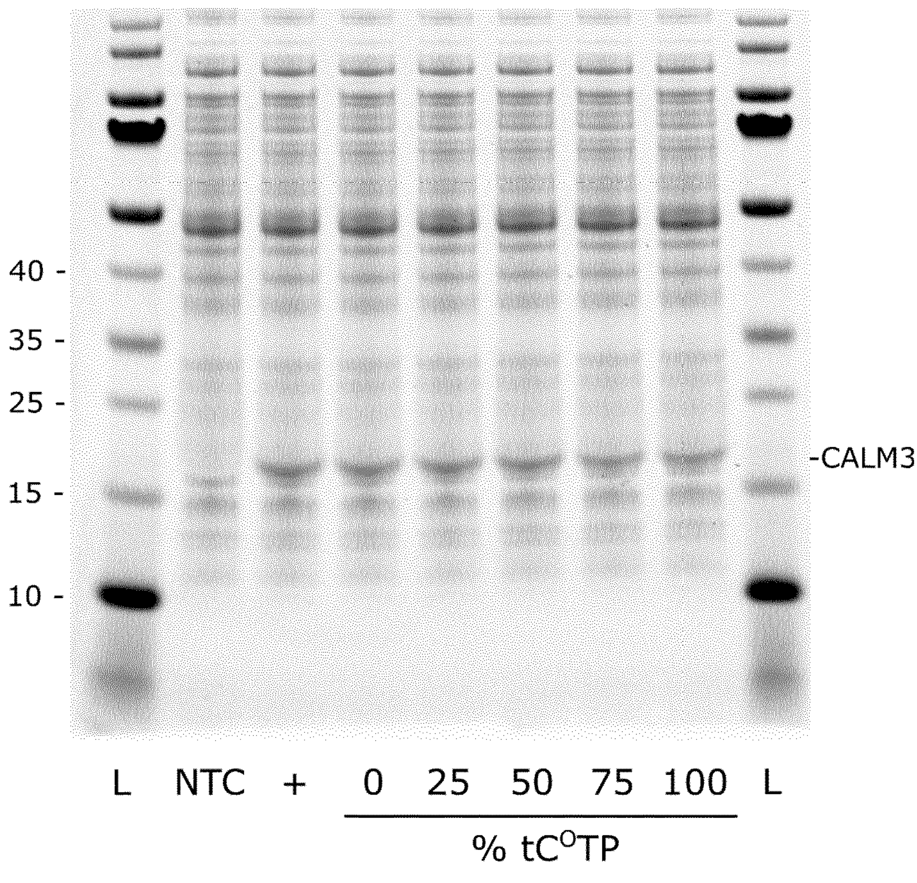

Figure 6b

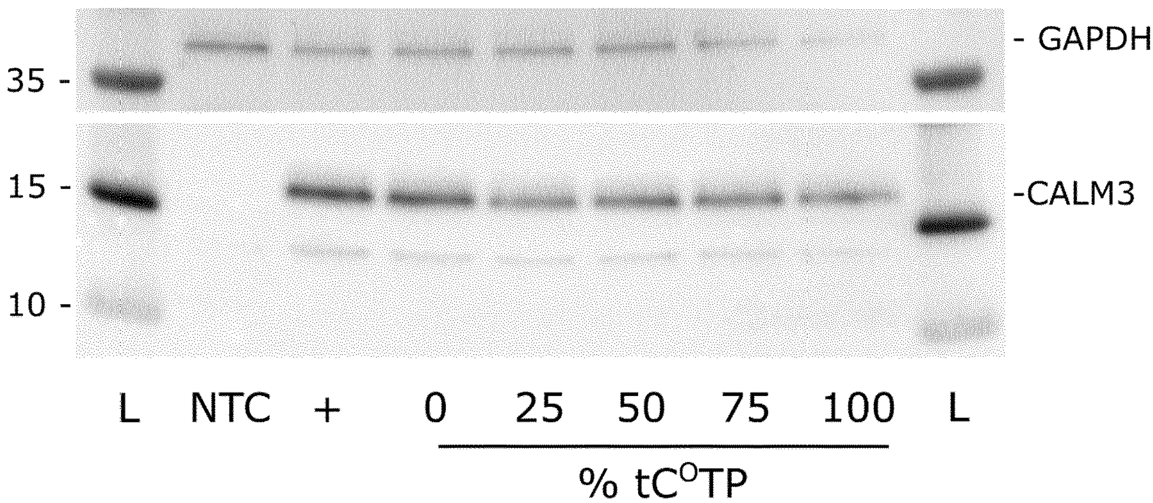

Figure 6c

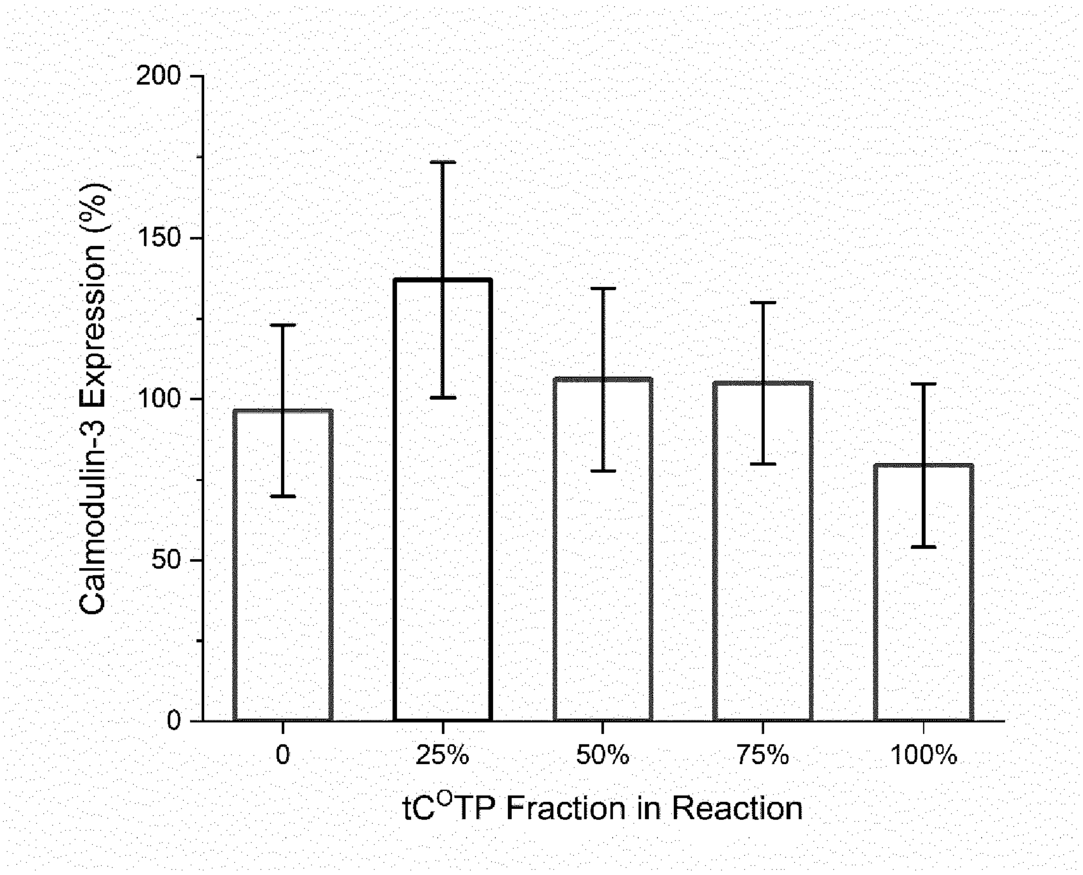
200
150
100
50
0
Calmodulin-3 Expression (%)
0
25%
50%
75%
100%
tC$^{O}$TP Fraction in Reaction

Figure 7a

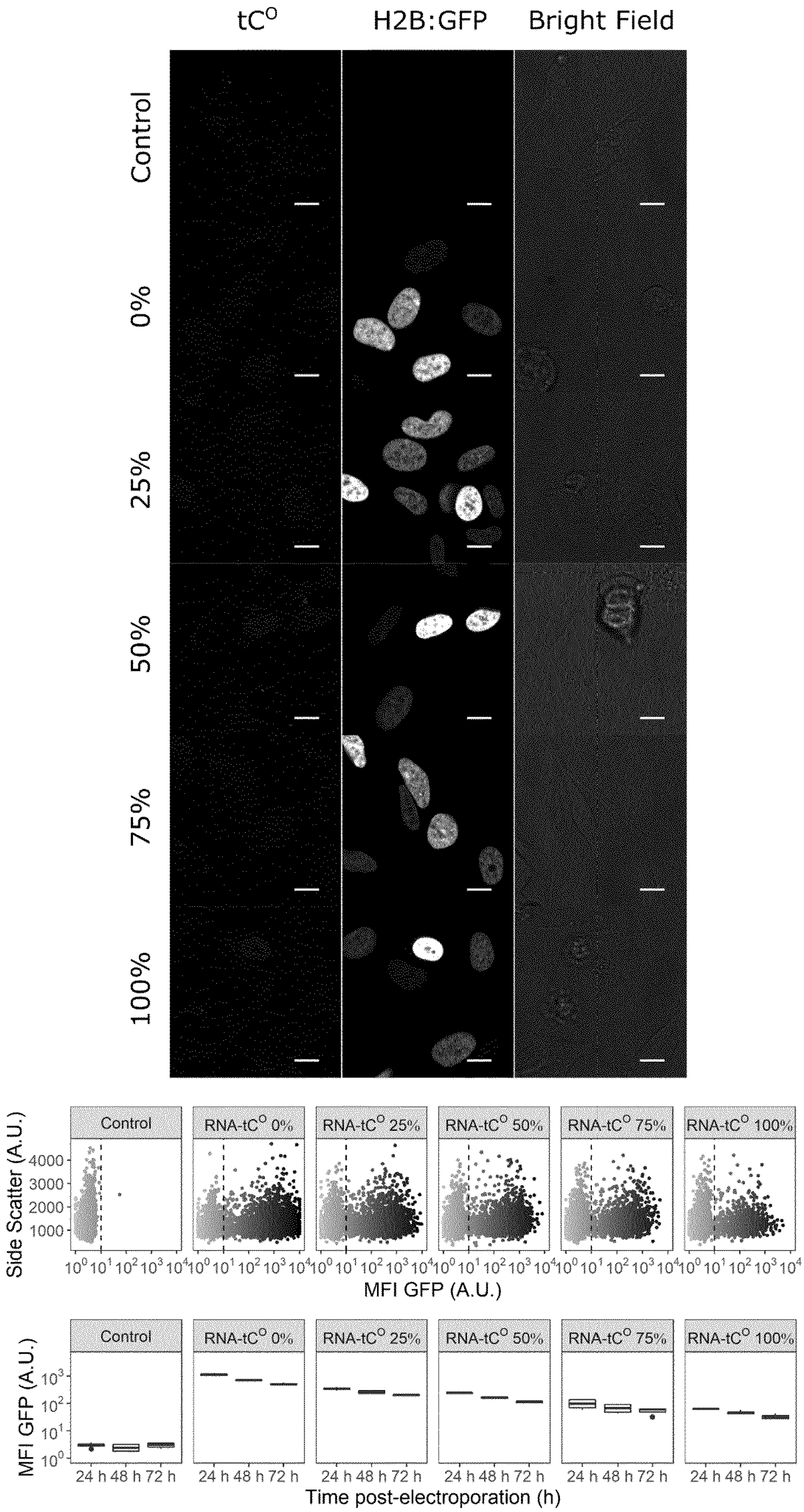
tC$^{O}$
H2B:GFP
Bright Field
Control
0%
25%
50%
75%
100%
Control
RNA-tC$^{O}$ 0%
RNA-tC$^{O}$ 25%
RNA-tC$^{O}$ 50%
RNA-tC$^{O}$ 75%
RNA-tC$^{O}$ 100%
Side Scatter (A.U.)
MFI GFP (A.U.)
MFI GFP (A.U.)
Time post-electroporation (h)
24 h 48 h 72 h

Figure 7b

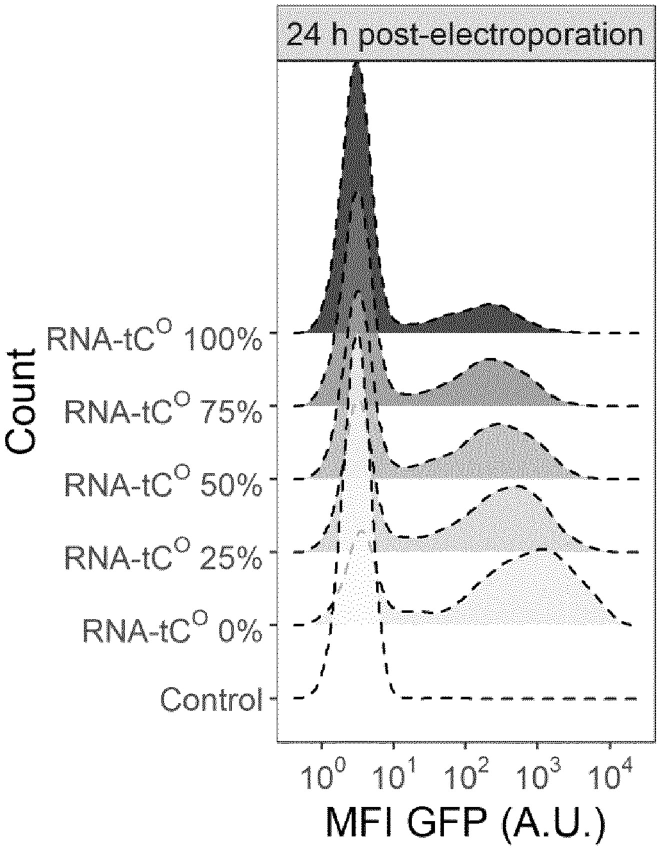

24 h post-electroporation
Count
RNA-tC<sup>O</sup> 100%
RNA-tC<sup>O</sup> 75%
RNA-tC<sup>O</sup> 50%
RNA-tC<sup>O</sup> 25%
RNA-tC<sup>O</sup> 0%
Control
$10^0$ $10^1$ $10^2$ $10^3$ $10^4$
MFI GFP (A.U.)

Figure 7c

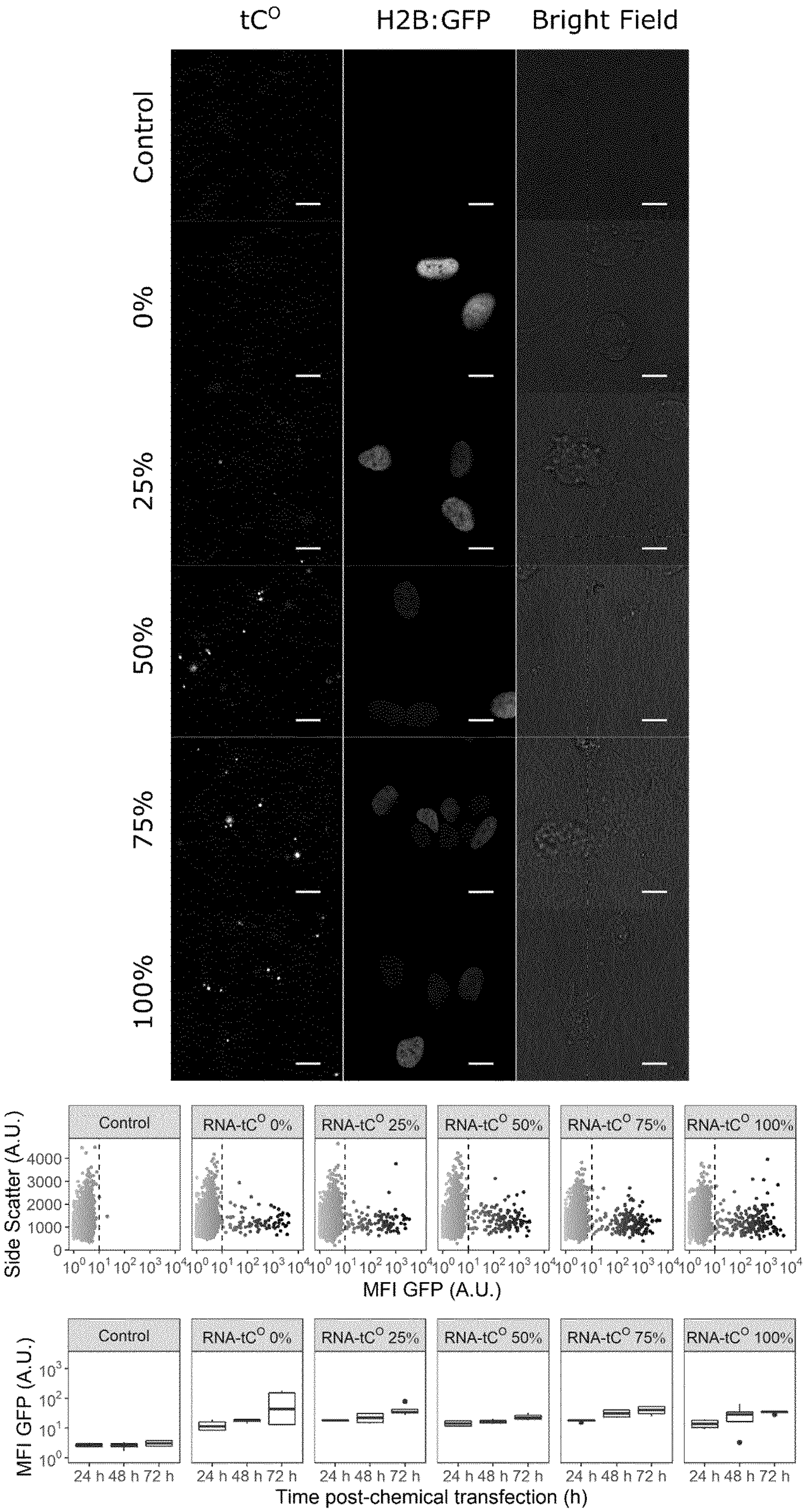
tC^O
H2B:GFP
Bright Field
Control
0%
25%
50%
75%
100%
Control
RNA-tC^O 0%
RNA-tC^O 25%
RNA-tC^O 50%
RNA-tC^O 75%
RNA-tC^O 100%
Side Scatter (A.U.)
MFI GFP (A.U.)
MFI GFP (A.U.)
24 h 48 h 72 h
Time post-chemical transfection (h)

**Figure 7d**

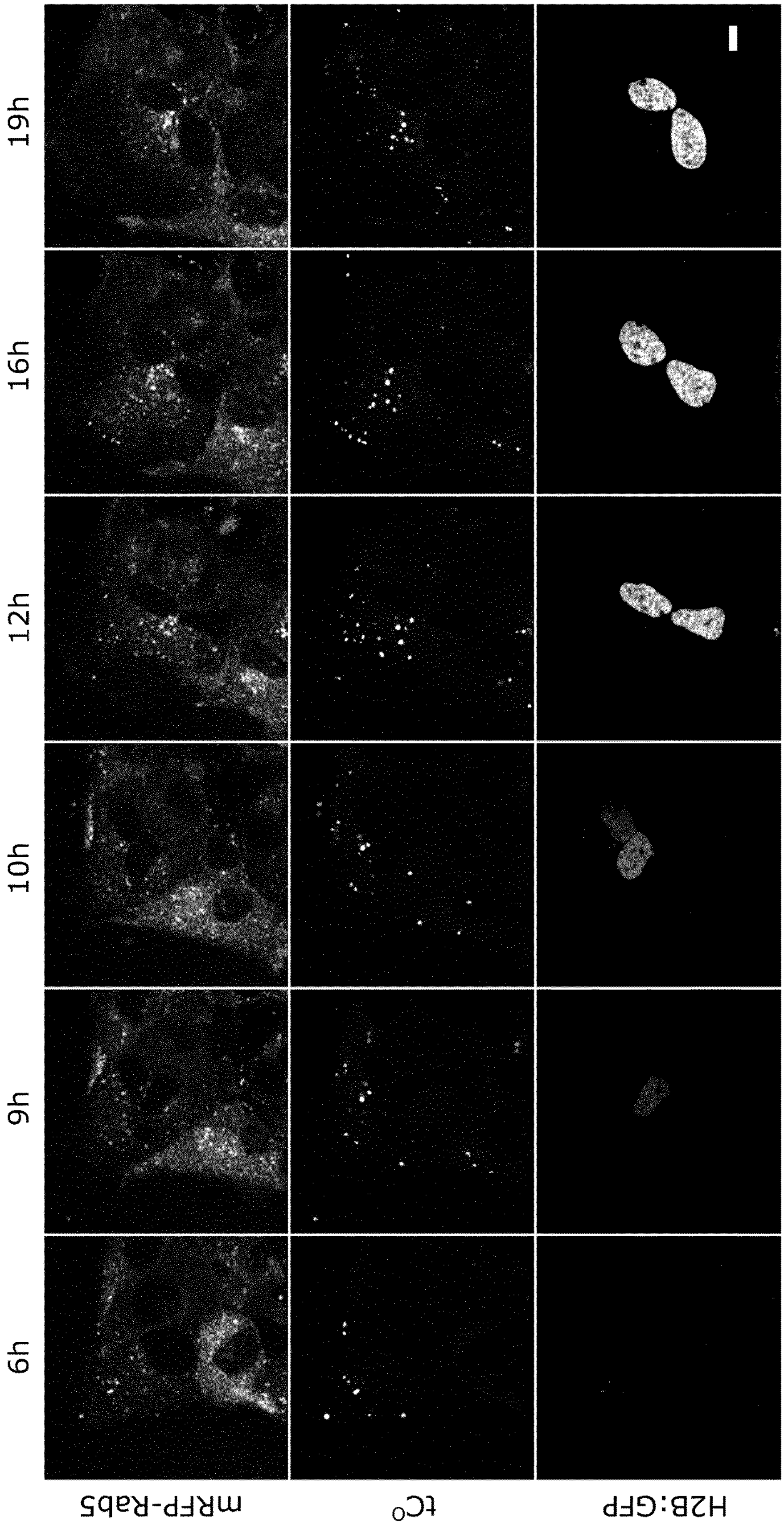

Figure 7e

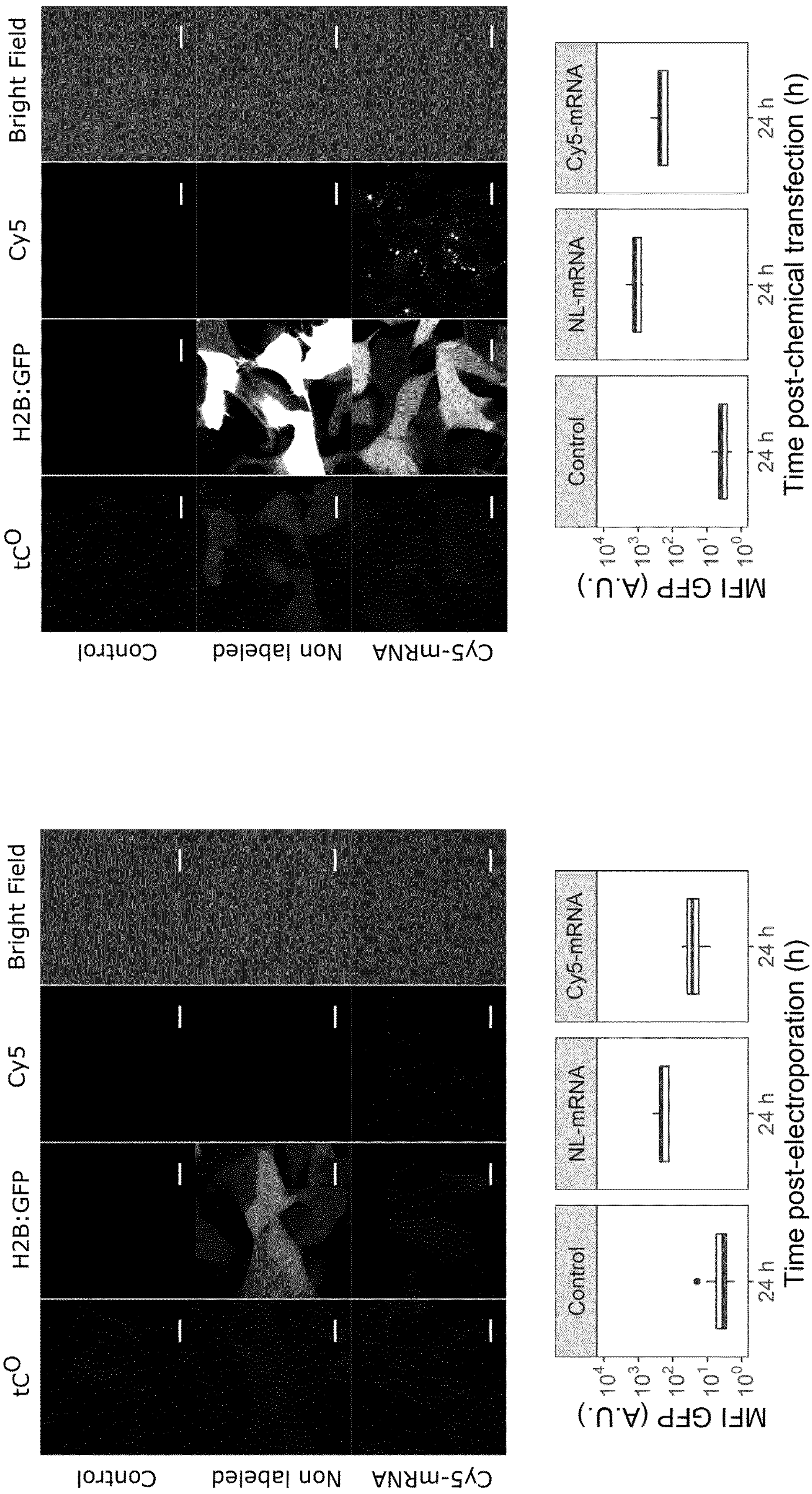
tC^O
H2B:GFP
Cy5
Bright Field
Control
Non labeled
Cy5-mRNA
MFI GFP (A.U.)
10^4
10^3
10^2
10^1
10^0
NL-mRNA
24 h
Time post-chemical transfection (h)
Time post-electroporation (h)

Figure 7f

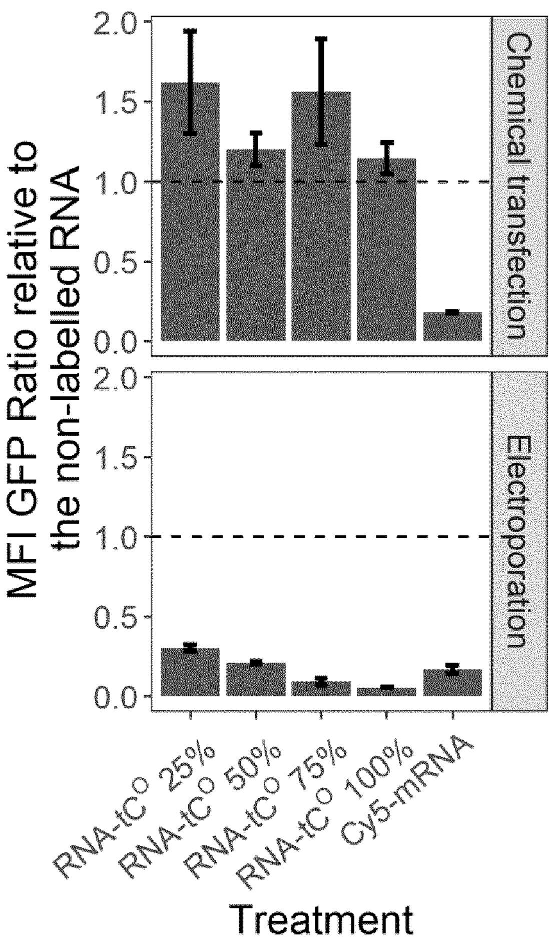
MFI GFP Ratio relative to the non-labelled RNA
2.0
1.5
1.0
0.5
0.0
Chemical transfection
Electroporation
RNA-tC$^{O}$ 25%
RNA-tC$^{O}$ 50%
RNA-tC$^{O}$ 75%
RNA-tC$^{O}$ 100%
Cy5-mRNA
Treatment

Figure 8a

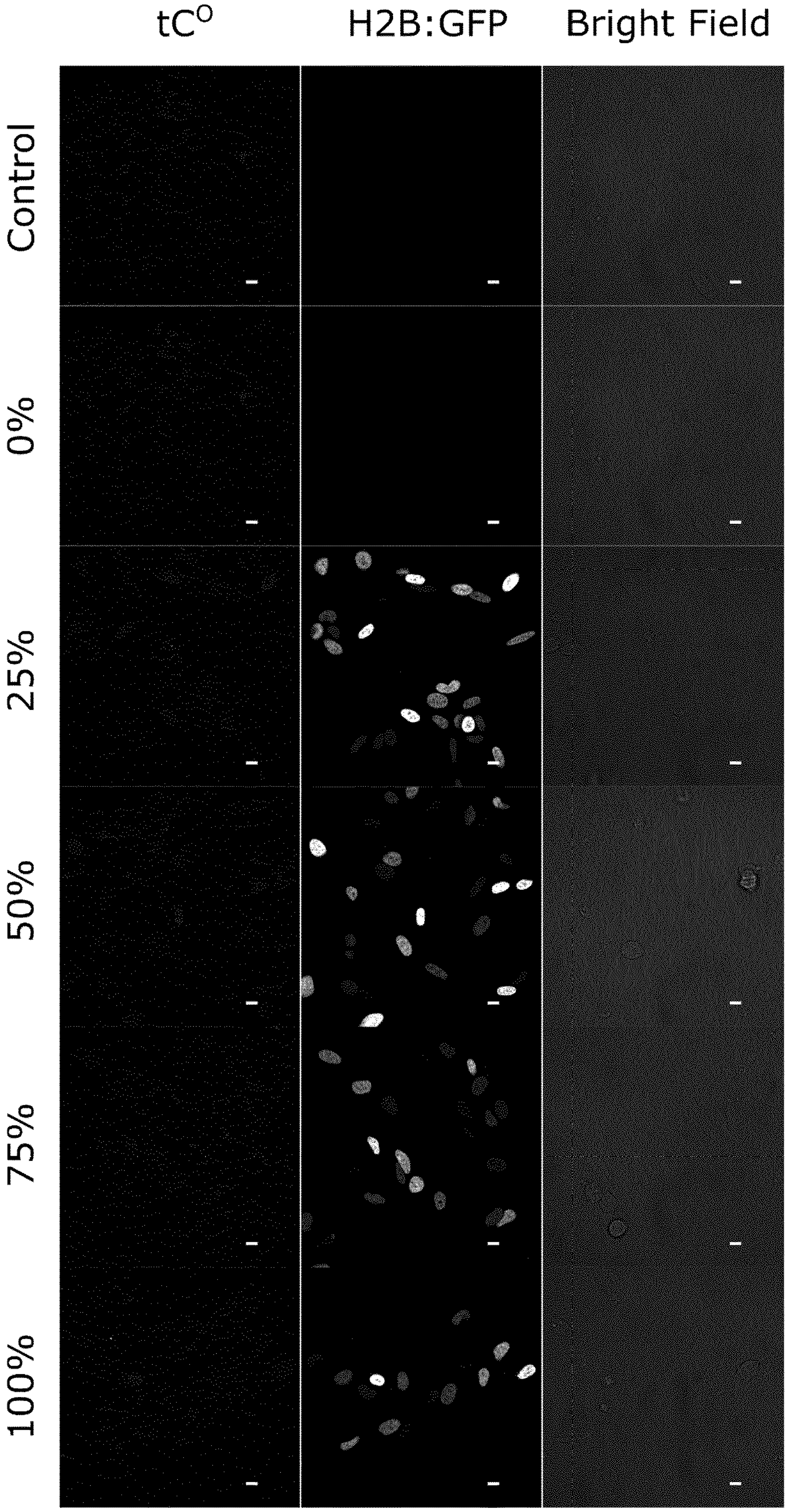

Figure 8b

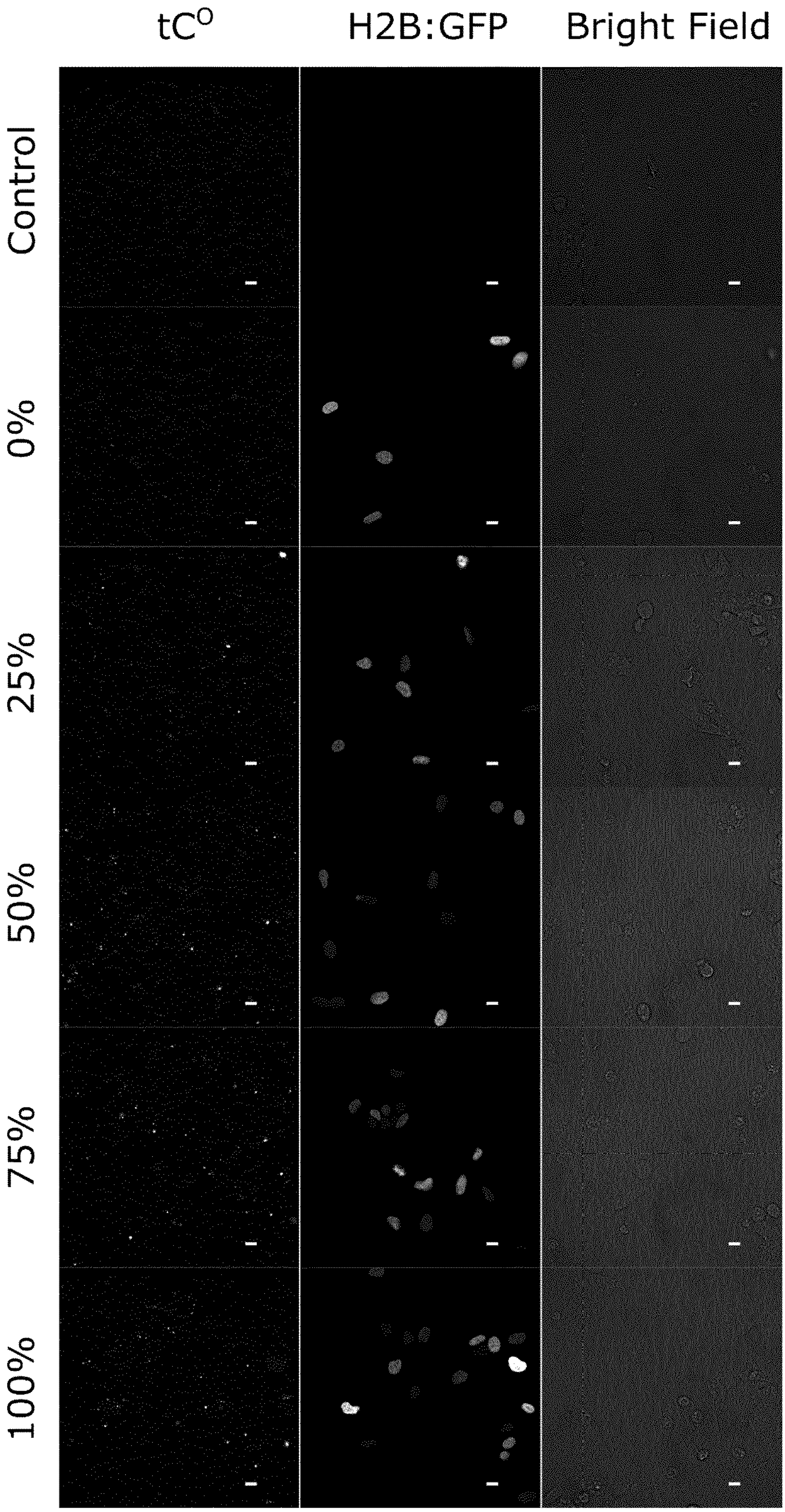

Figure 8c

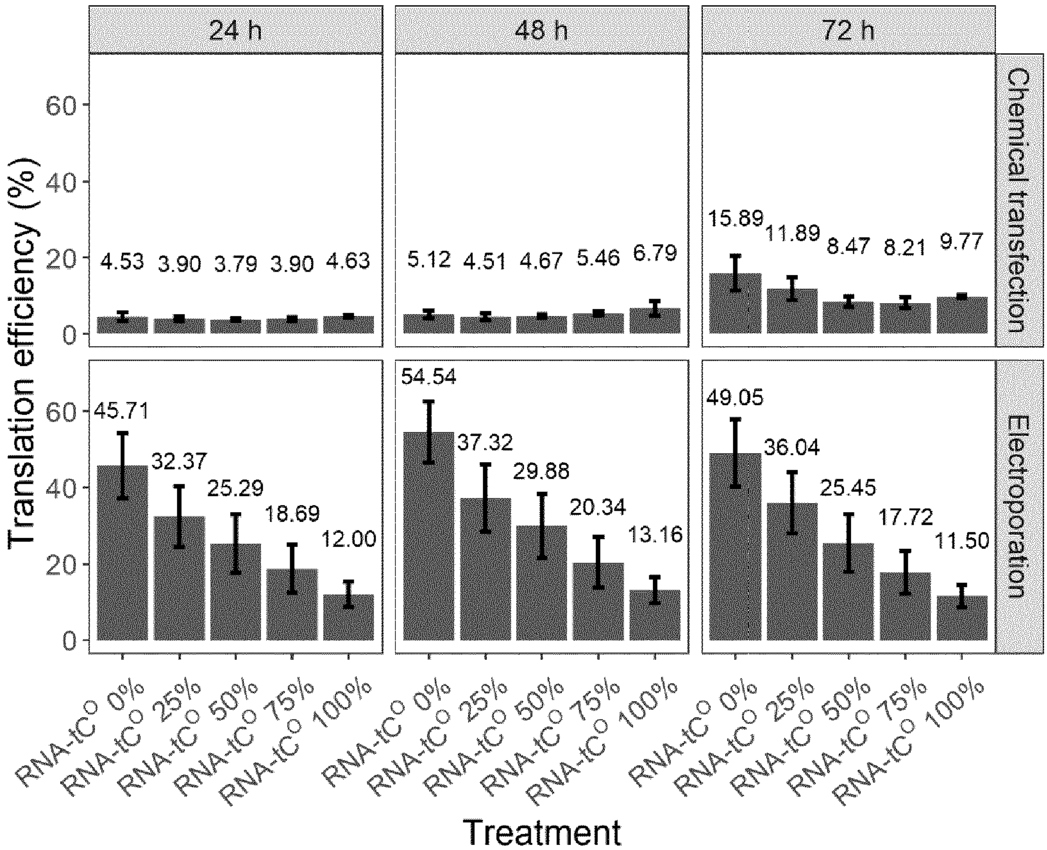
24 h
48 h
72 h
Chemical transfection
Electroporation
Translation efficiency (%)
0
20
40
60
4.53 3.90 3.79 3.90 4.63
5.12 4.51 4.67 5.46 6.79
15.89 11.89 8.47 8.21 9.77
45.71 32.37 25.29 18.69 12.00
54.54 37.32 29.88 20.34 13.16
49.05 36.04 25.45 17.72 11.50
RNA-tCO 0%
RNA-tCO 25%
RNA-tCO 50%
RNA-tCO 75%
RNA-tCO 100%
Treatment

**Figure 8d**

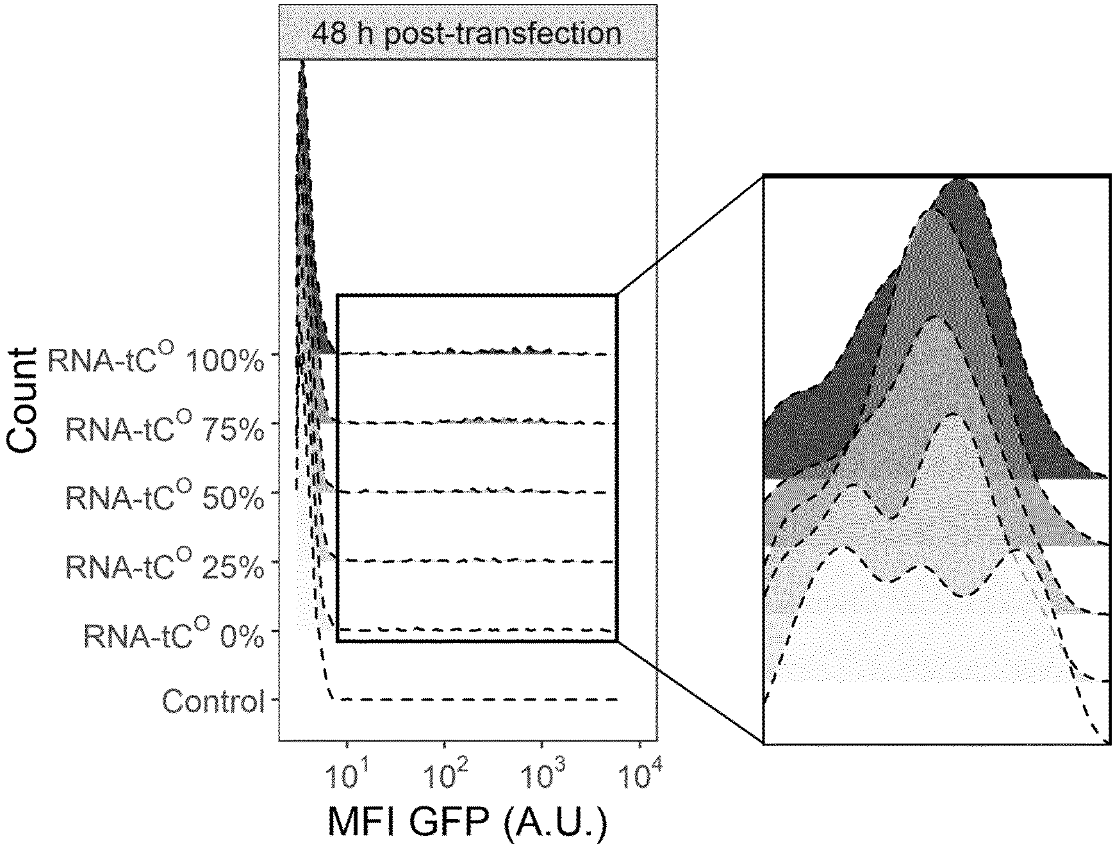
48 h post-transfection
Count
RNA-tC$^O$ 100%
RNA-tC$^O$ 75%
RNA-tC$^O$ 50%
RNA-tC$^O$ 25%
RNA-tC$^O$ 0%
Control
$10^1$ $10^2$ $10^3$ $10^4$
MFI GFP (A.U.)

Figure 8e

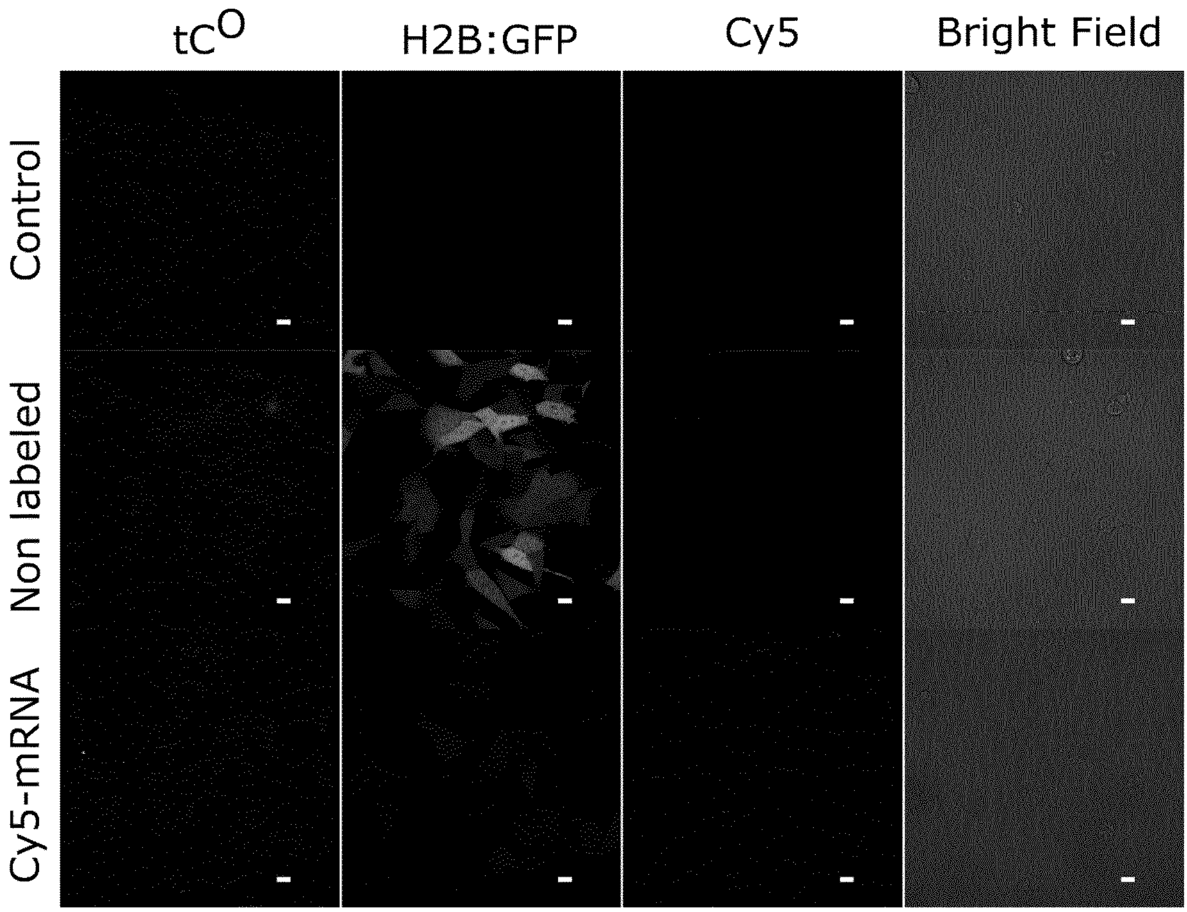

Figure 8f

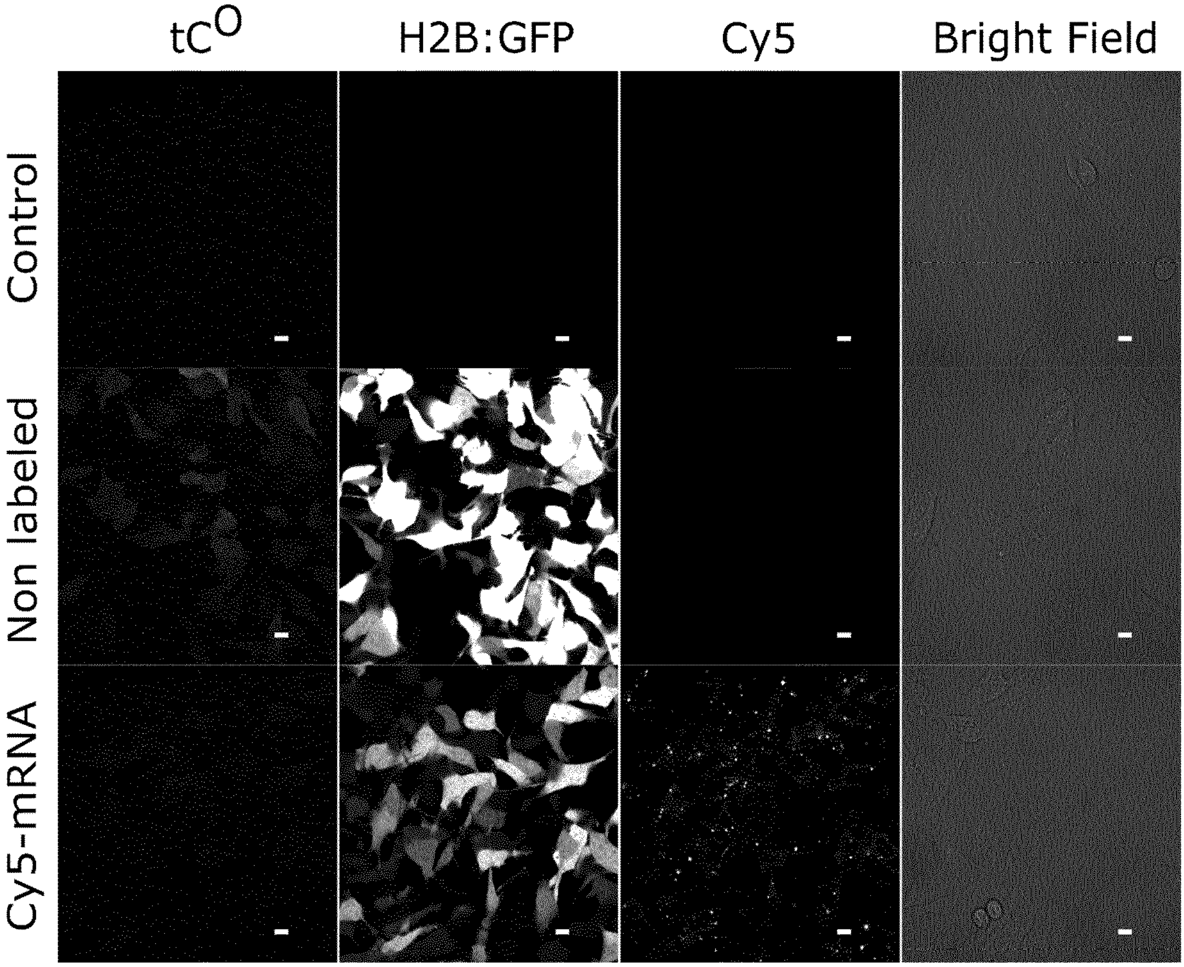

Figure 8g

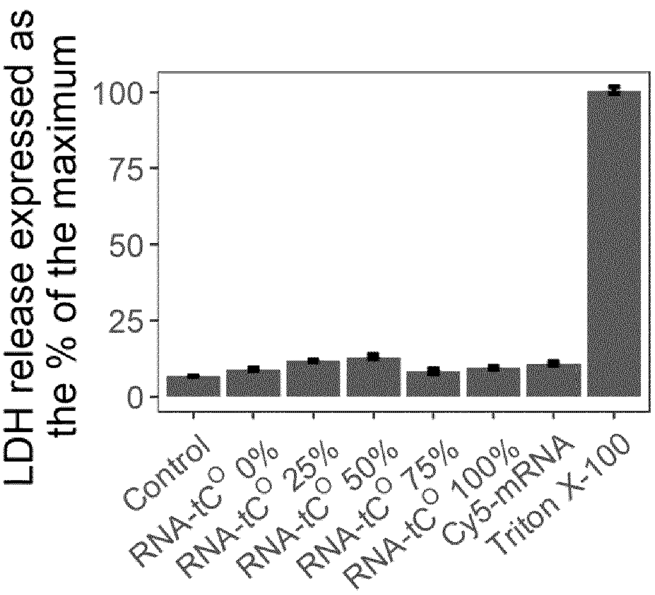
LDH release expressed as the % of the maximum
100
75
50
25
0
Control
RNA-tC° 0%
RNA-tC° 25%
RNA-tC° 50%
RNA-tC° 75%
RNA-tC° 100%
Cy5-mRNA
Triton X-100

Figure 8h

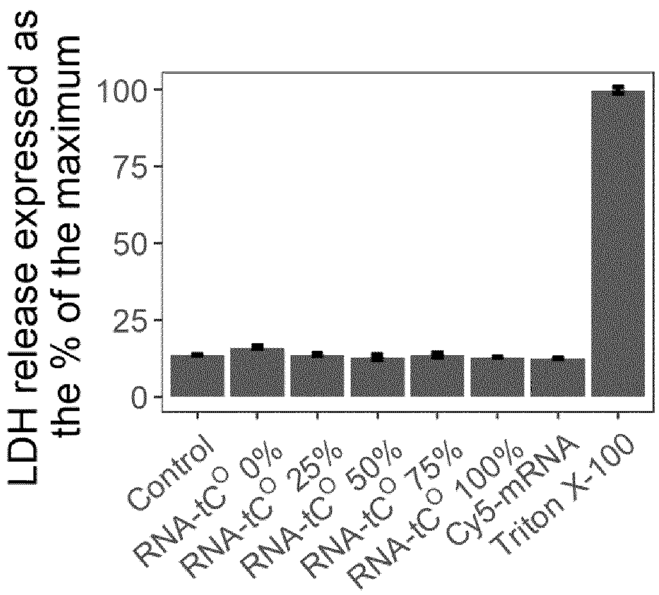
LDH release expressed as
the % of the maximum
100
75
50
25
0
Control
RNA-tC$^{O}$ 0%
RNA-tC$^{O}$ 25%
RNA-tC$^{O}$ 50%
RNA-tC$^{O}$ 75%
RNA-tC$^{O}$ 100%
Cy5-mRNA
Triton X-100

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011034895 A


### Non-patent literature cited in the description


- **DOWDY, S. F.** *Nat. Biotechnol.,* 2017, vol. 35, 222-229
- **CROOKE, S. T. et al.** *Not . Biotechnol.,* 2017, vol. 35, 230
- **PEI, D ; BUYANOVA, M.** *Bioconjugate Chem.,* 2018
- **LI, Y. ; KE, K. ; SPITALE, R. C.** *Biochemistry,* 2019, vol. 58, 379-386
- **WILHELMSSON, M. et al.** *Sci. Rep.,* 2017, vol. 7, 2393
- **GREENE T. W. ; WUTS P. G. M.** Greene's Protective Groups in Organic Synthesis. John Wiley & Sons, Inc, 2007
- **DUCHO, C. et al.** *J . Med . Chem.,* 2007, vol. 50, 1335-1346
- **WARNECKE, S. ; MEIER, C.** *J. Org. Chem.,* 2009, vol. 74, 3024-3030
- **THOMEN, P. et al.** *Biophys. J.,* 2008, vol. 95, 2423-2433
- **ARANDA, P. S ; LAJOIE, D. M. ; JORCYK, C. L.** *Electrophoresis,* 2012, vol. 33, 366-369